# EUROPEAN PATENT APPLICATION

(11) **EP 4 825 446 A2**
(43) Date of publication of application: **21.10.2026**
(21) Application number: 26203306.1
(22) Date of filing: 24.01.2023
(51) Int. Cl.: A61P 31/04

(54) **VACCINE AGAINST LEPTOSPIROSIS**

(30) Priority: 25.01.2022 US 202263302817 P
(62) Document number(s) of the earlier application(s) in accordance with Art. 76 EPC: 23747788.0 / 4 469 470
(71) Applicant: Yale University, New Haven CT 06510 (US)
(72) Inventor: WUNDER, Elsio, New Haven 06511 (US); KO, Albert, New Haven 06511 (US)
(74) Representative: Kilburn & Strode LLP

(57) **Abstract**

The disclosure provides a composition comprising an effective amount of a plurality of leptospiral proteins, or immunogenic fragments thereof. The disclosure further provides a method of generating an immune response against leptospirosis using a composition comprising the plurality of leptospiral proteins, or immunogenic fragments thereof, and an adjuvant.

## Description

### CROSS-REFERENCE TO RELATED APPLICATIONS

This application claims priority under 35 U.S.C. § 119(e) to U.S. Provisional Patent Application No. 63/302,817, filed January 25, 2022, which is incorporated herein by reference in its entirety.

### STATEMENT OF RIGHTS TO INVENTIONS MADE UNDER FEDERALLY SPONSORED RESEARCH

This invention was made with government support under AI088752 and AI121207 awarded by National Institutes of Health. The government has certain rights in the invention.

### BACKGROUND OF THE INVENTION

Leptospirosis is a life-threatening disease, which can occur in a diverse range of epidemiological situations. Current leptospirosis control measures have been uniformly ineffective in addressing the large human and animal health burden due to leptospirosis worldwide. The major challenge has been developing a vaccine that protects against all the 38 genetically diverse pathogenic species and >300 serovars which are potential agents for leptospirosis. All attempts to identify vaccine candidates that can be used as a widely-applicable vaccine against leptospirosis have failed.

Therefore, a need exists in the art for an effective leptospirosis vaccine that is protective against a wide variety of genetically diverse pathogenic species and serovars. The present invention addresses this need.

### SUMMARY OF THE INVENTION

In one aspect, the invention provides a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

In another aspect, the invention provides a method of conditioning an immune response against leptospirosis in a subject, the method comprising administering to the subject an effective amount of a composition comprising a plurality of leptospira peptides, each leptospira peptide having 80% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.

In various embodiments, each leptospira peptide has 90% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.

In various embodiments, each leptospira peptide has 95% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.

In various embodiments, the composition further comprises an adjuvant.

In various embodiments, the composition further comprises a pharmaceutically acceptable carrier.

In various embodiments, the composition comprises a plurality of leptospira peptides comprising a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4.

In various embodiments, the composition comprises a plurality of leptospira peptides comprising a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8.

In various embodiments, the composition comprises a plurality of leptospira peptides comprising a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6.

In various embodiments, the composition is administered to the subject in three separate doses.

In various embodiments, the subject is a mammal.

In various embodiments, the subject is a human.

### BRIEF DESCRIPTION OF THE DRAWINGS

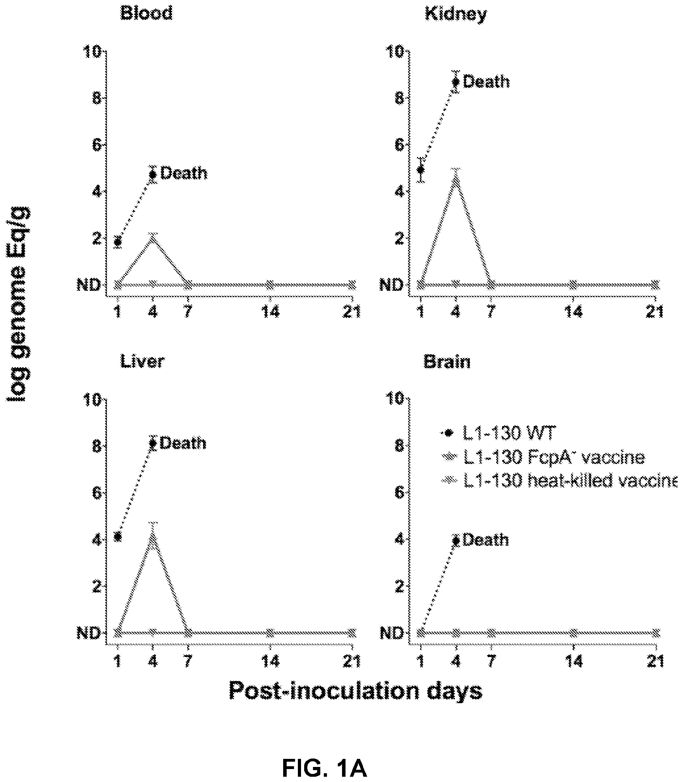

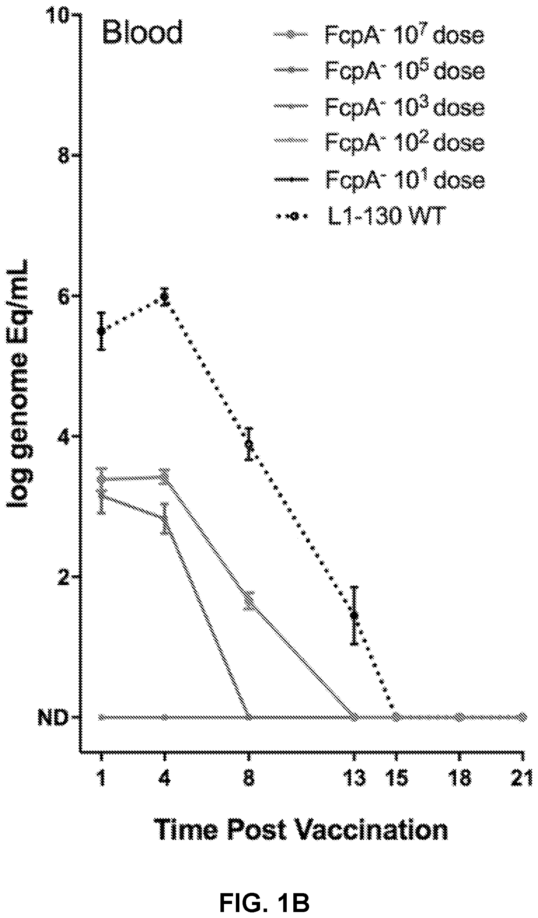

FIGs. 1A-1B depict the dissemination of the L1-130 *fcpA⁻* mutant in animal tissues. FIG. 1A: Kinetics of infection of L1-130 WT, L1-130 *fcpA-* vaccine, and L1-130 heat-killed vaccine in blood, kidney, liver, and brain of hamsters after inoculation with 10⁷ bacteria. All animals infected with WT strain died between 5 and 6 days post-infection. FIG. 1B: Kinetics of infection of L1-130 WT (10⁷ leptospires) and L1-130 *fcpA⁻* attenuated-vaccine (dose range from 10⁷ to 10¹ leptospires) in blood of mouse. Results are expressed by logarithmic genome equivalent per gram or milliliter of tissues with mean and standard deviation. All doses were inoculated by subcutaneous route in both models.

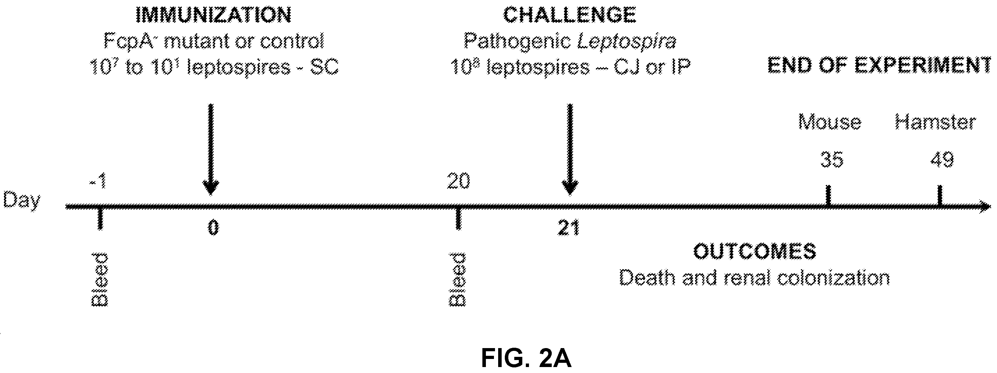

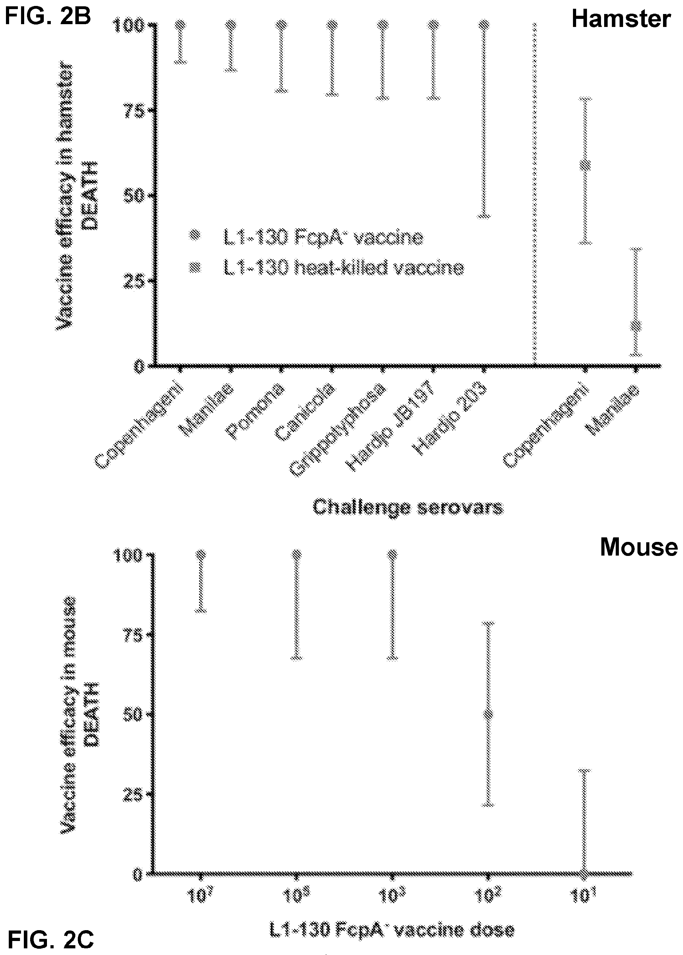

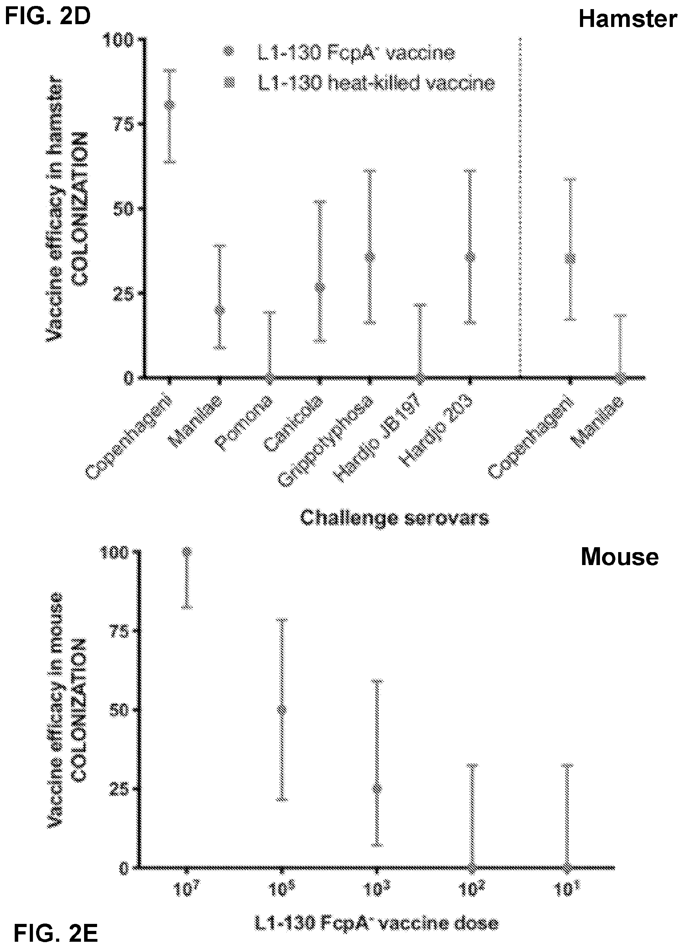

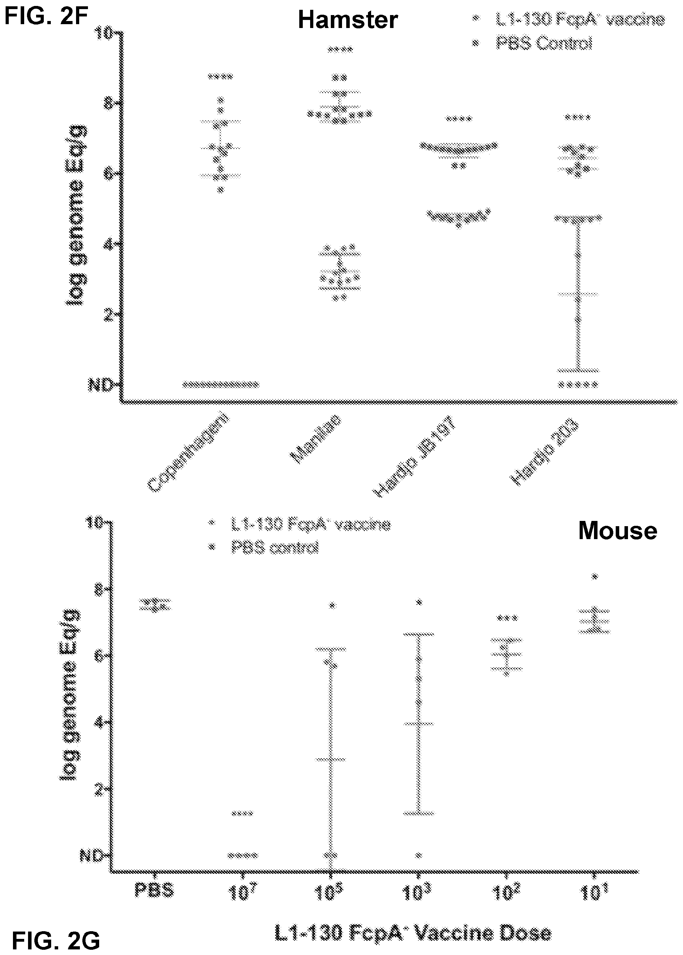

FIGs. 2A-2G depict the efficacy of the L1-130 *fcpA⁻* attenuated vaccine model. Animals were vaccinated with a dose of 10⁷ leptospires (hamsters) or a range of doses from 10⁷ to 10¹ leptospires (mice) by subcutaneous (SC) route. Animals were bled the day before immunization (day -1) and day 20 post-immunization (FIG. 2A). Hamsters were challenged by conjunctival route with either the homologous strain or different heterologous strains. Mice were challenged by intraperitoneal route with the heterologous serovar Manilae of L. interrogans. By combining all vaccine experiments performed, efficacy of the vaccine against death and colonization was evaluated for hamsters (FIG. 2B and FIG. 2D) and mice (FIG. 2C and FIG. 2E) and represented by percentage and 95% CI based on frequency of outcomes compared to PBS-immunized animals. Hamster experiment are showing the results after vaccination with the *fcpA⁻* attenuated-vaccine (red) and heat-killed vaccine (blue). Bacterial load in the kidney was measured by qPCR in hamsters (FIG. 2F) and mice (FIG. 2G) and compared between PBS-immunized animals (blue) and animals immunized with fcpA^{~} attenuated-mutant (red). Results are expressed in logarithmic genome equivalents per gram of renal tissue with mean and standard deviation. Asterisk symbols represent statistical significance calculated by t-test: *p<0.01, ***p<0.0001. See also Tables 3 and 4.

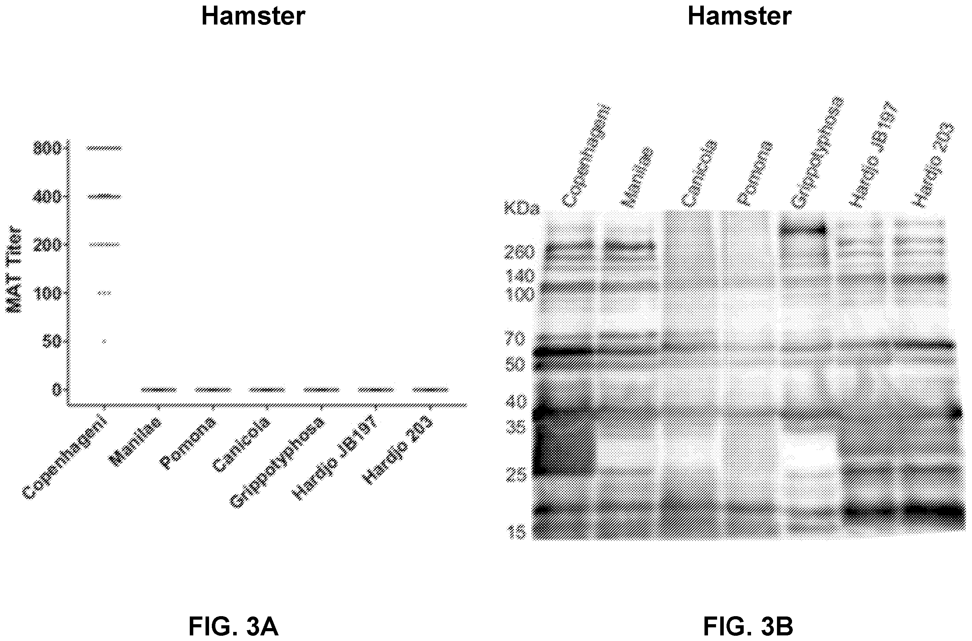

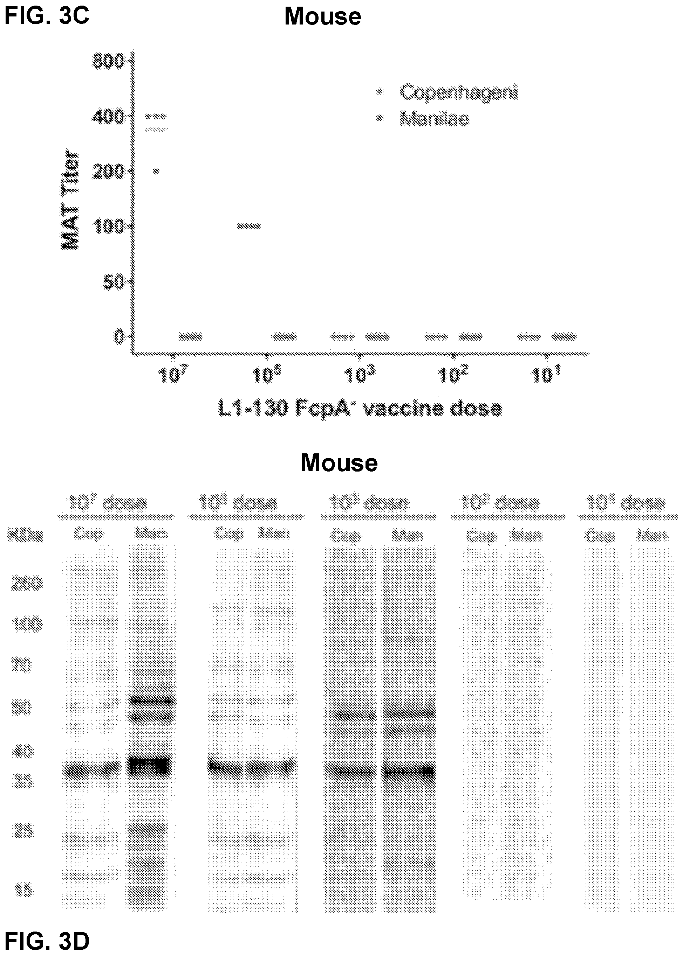

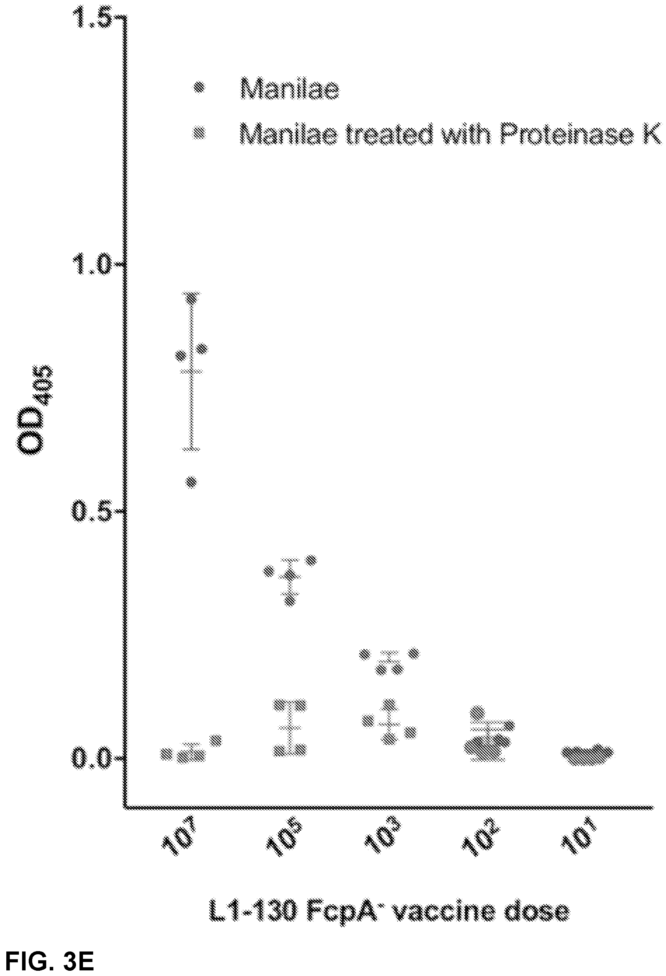

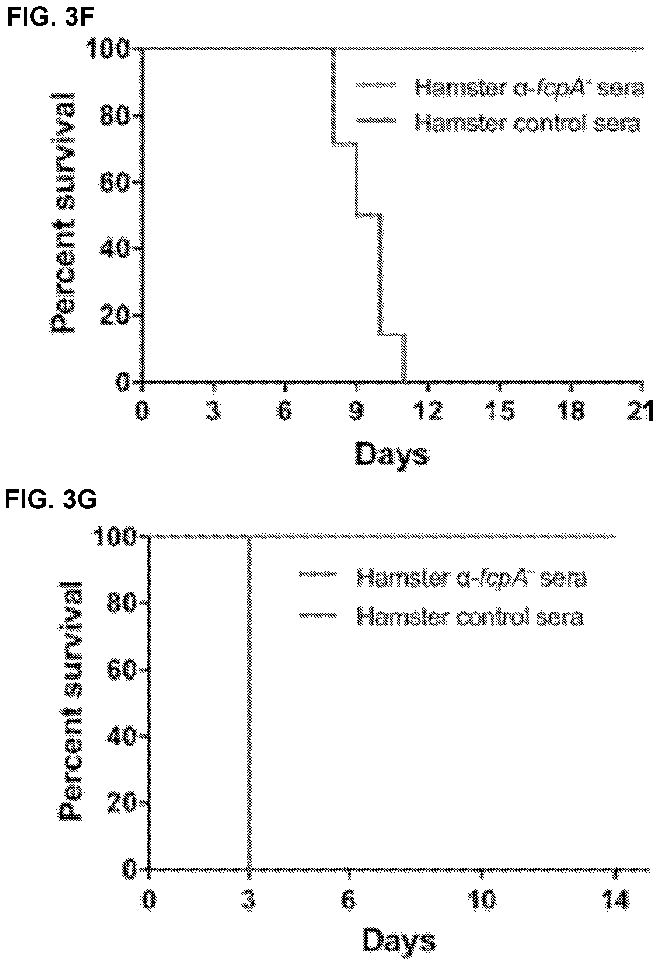

FIGs. 3A-3G depict the immunogenicity and correlates of immunity for the L1-130 *fcpA⁻* attenuated vaccine model. Individual sera of hamsters and mice were obtained after 20 days post-vaccination by a subcutaneous (SC) dose of 10⁷ leptospires (hamsters) or a range of doses from 10⁷ to 10¹ leptospires (mice) of the attenuated-vaccine. Microscopic agglutination test (MAT) (FIG. 3A and FIG. 3C) and western blot (FIG. 3B and FIG. 3D) were performed adopting as antigen all the strains used for challenged in both hamster and mice, respectively. Mice sera was additionally tested using an ELISA assay (FIG. 3E) adopting whole-cell extract of serovar Manilae with (red) and without (blue) Proteinase K treatment as antigen. Furthermore, a pool of hamster immune-sera vaccinated with a dose of 10⁷ leptospires of *fcpA⁻* attenuated-vaccine was used for passive transfer experiments. 2 mL or 0.5 mL of sera was passively transfer to naïve hamsters (FIG. 3F) or mice (FIG. 3G), respectively, followed by challenge with a dose of 10⁸ leptospires of heterologous serovar Manilae by conjunctival (CJ) or intraperitoneal (IP) route, respectively. Results are expressed in a survival curve of animals passively transferred with *fcpA⁻*anti-sera (red) and control hamster sera (blue).

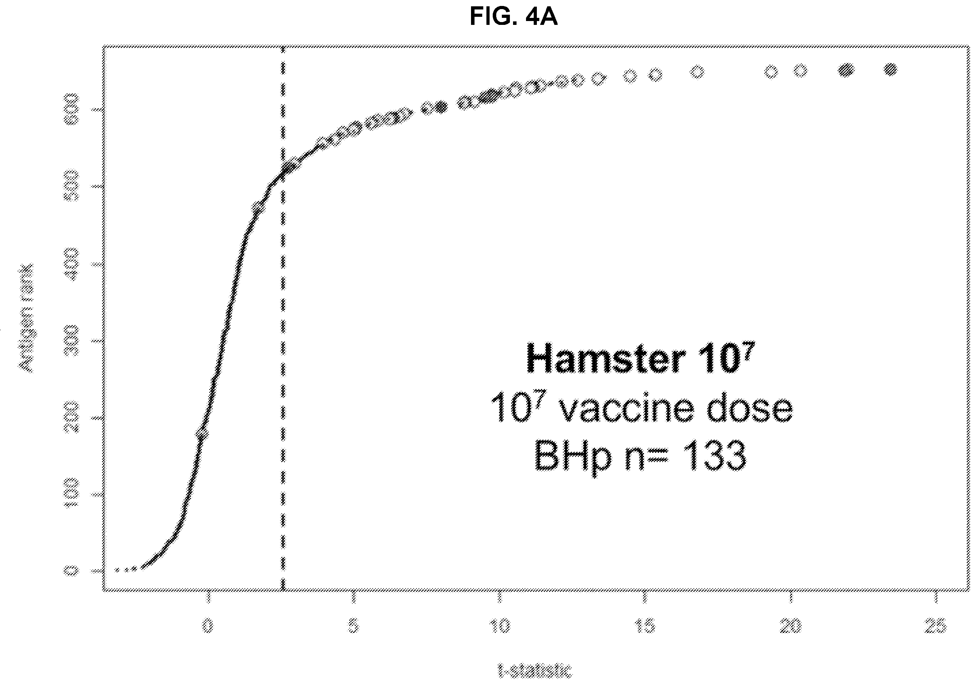

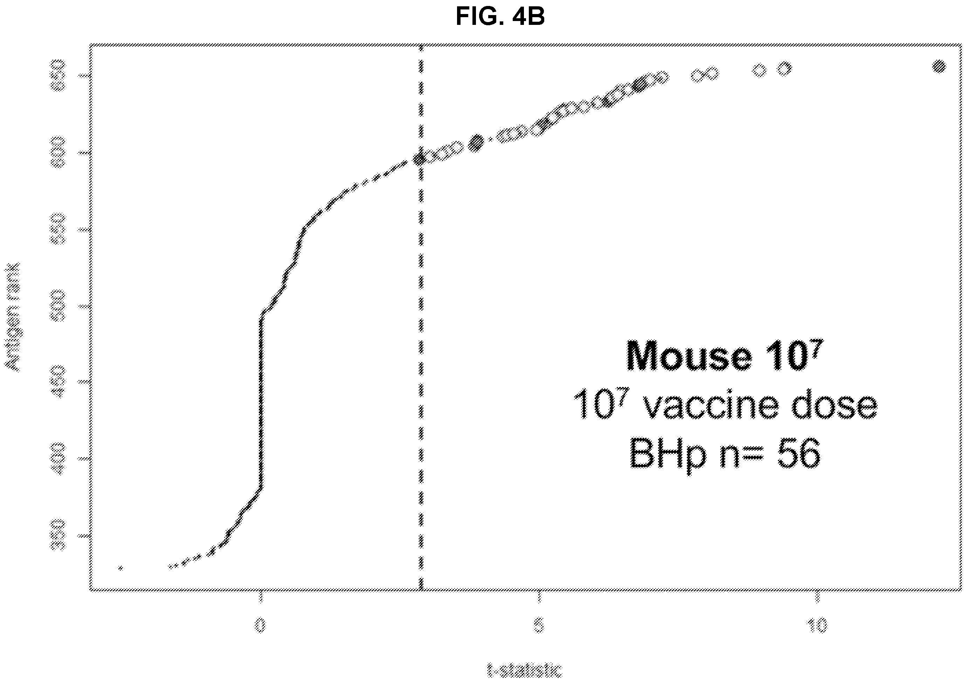

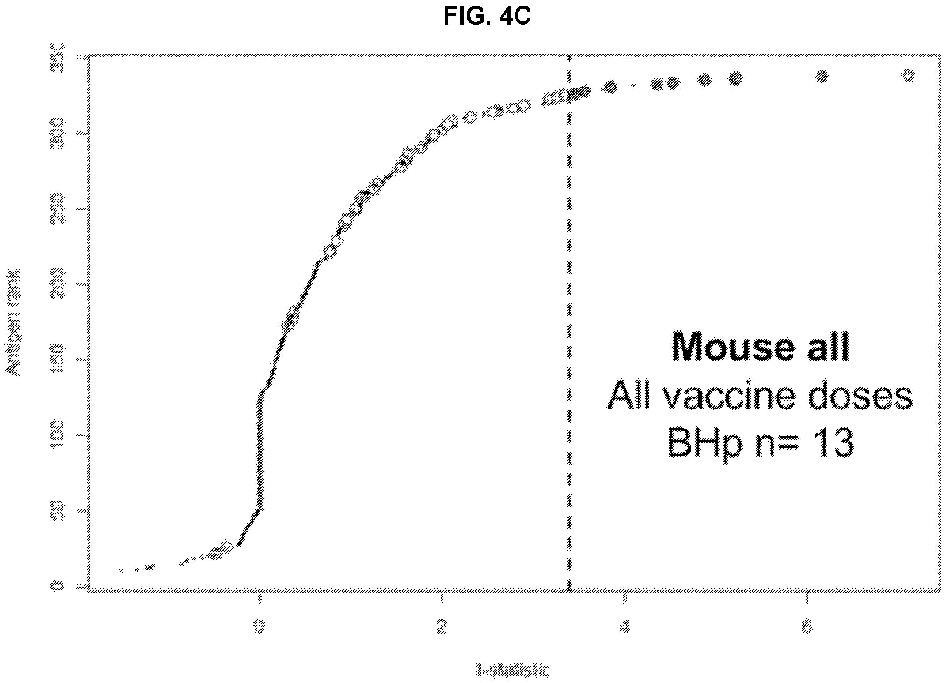

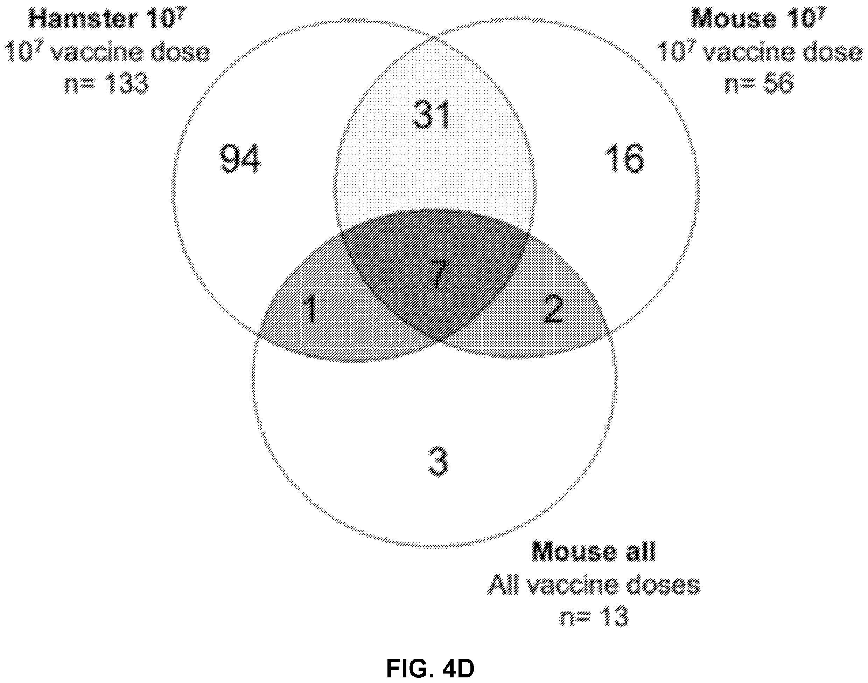

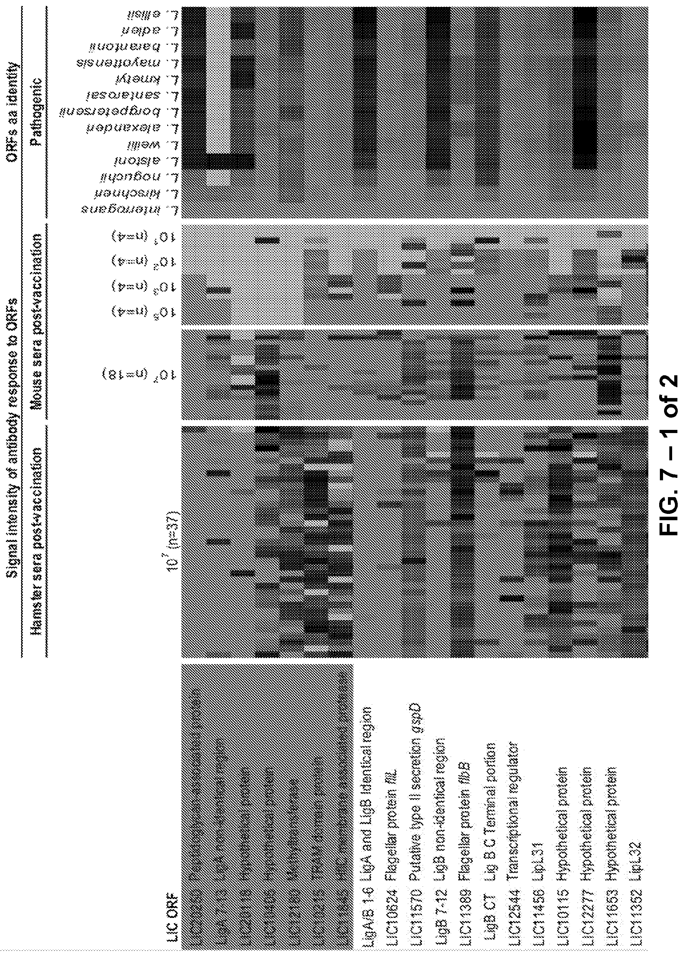

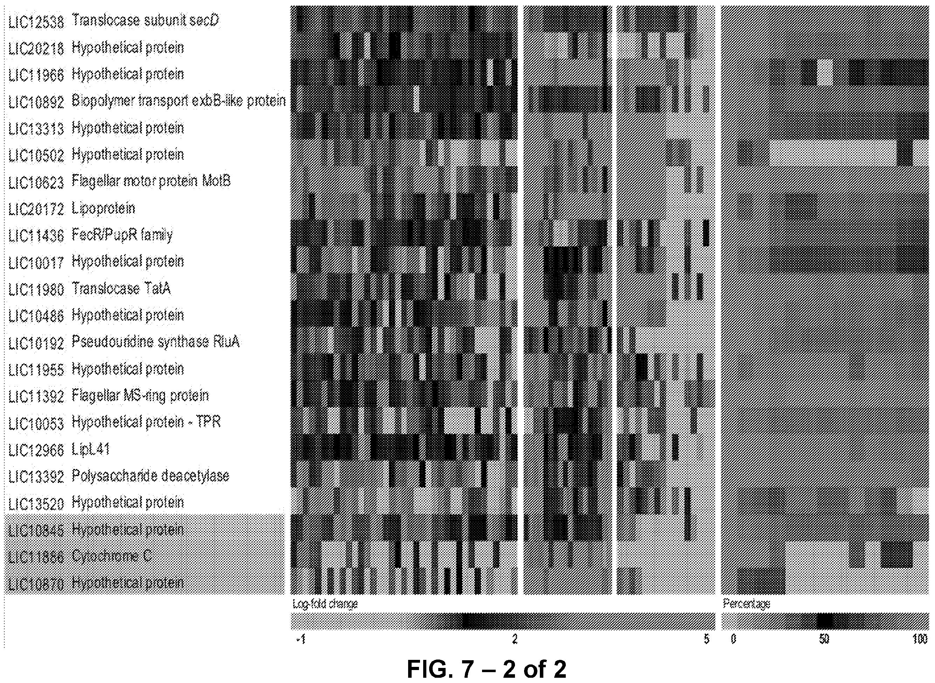

FIGs. 4A-4D depict the proteome array analysis of immune-sera against L1-130 *fcpA⁻* attenuated-vaccine. Using statistical modeling the t-statistics value was calculated for each individual antigen used in the proteome array (660 for hamster and 330 for mice) based on three groups: the contrast between vaccinated and unvaccinated hamsters (FIG. 4A) or mice (FIG. 4B) using a vaccine dose of 10⁷ leptospires; the dose-response relationship for each antigen on mice (FIG. 4C) vaccinated with a range of doses from 10⁷ to 10¹ leptospires of the attenuated-vaccine. Results are ranked based on individual t-statistics values for each antigen, and the dashed line represents the selection point for the antigens based on Bhp-test. The Venn-diagram (FIG. 4D) shows the relationship of all the 154 antigens identified in the three groups. The subgroups of antigens selected for further characterization are highlighted in color. See FIG. 7.

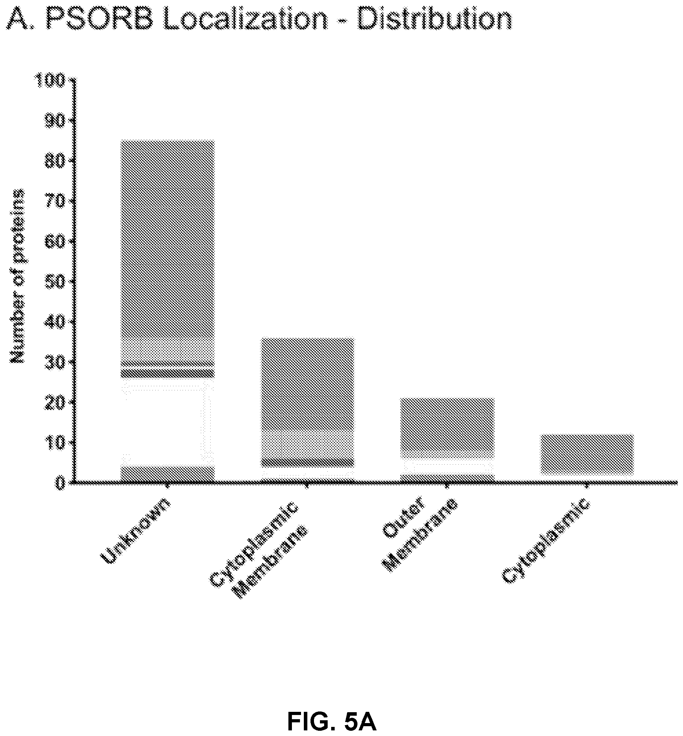

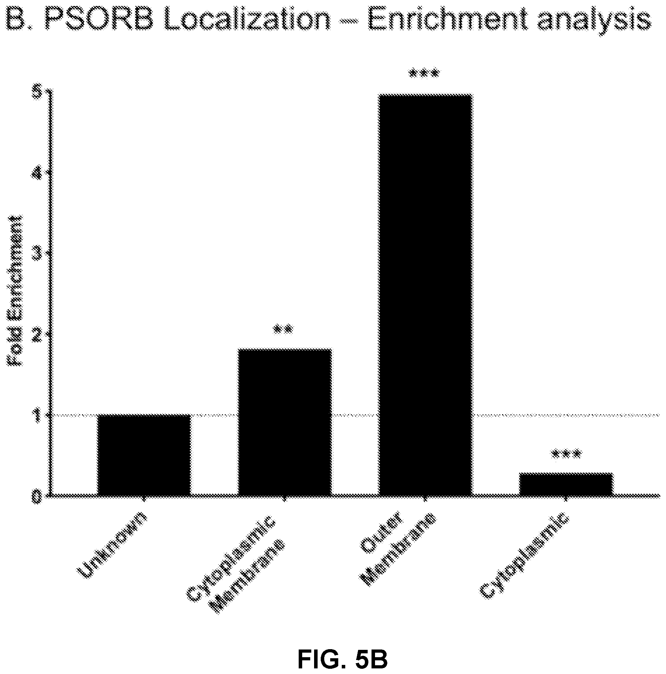

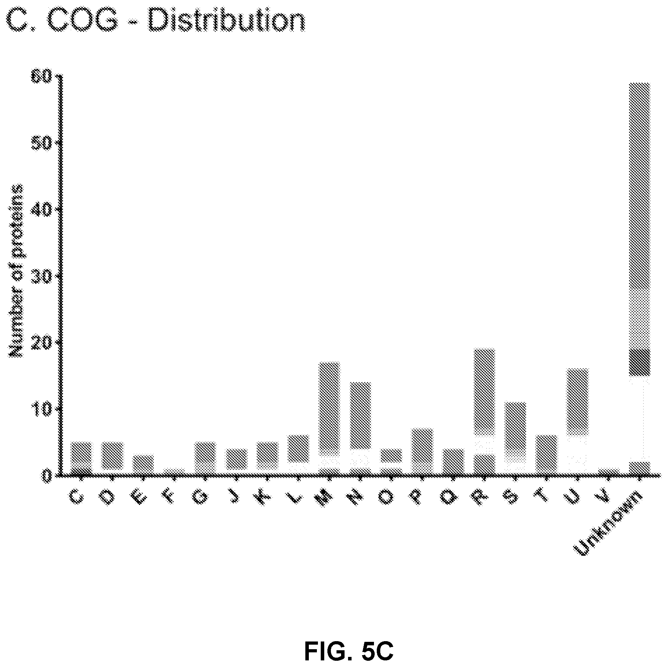

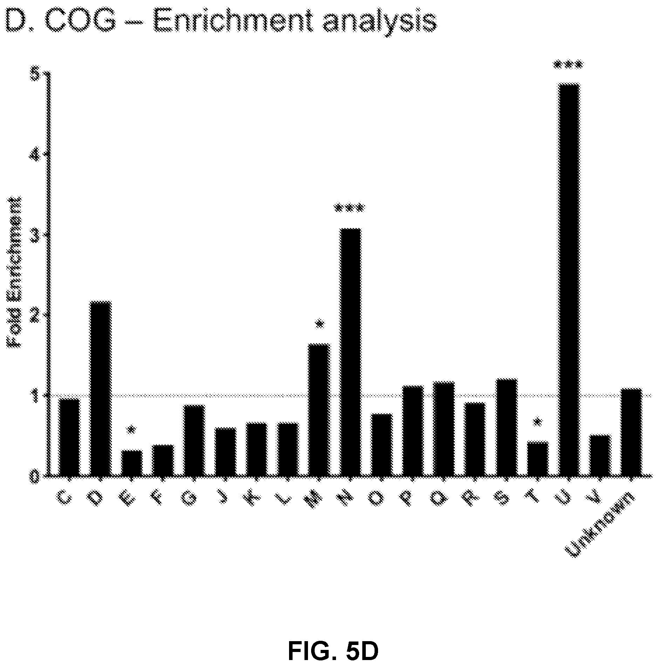

FIGs. 5A-5D depict the in silico analysis of the 154 protein targets. Using PSORB information and Genoscope database, the 154 protein targets identified in this study were classified by their putative localization in the cell (FIG. 5A) and their clusters of orthologous group (COG) classification (FIG. 5C). The groups of proteins are classified as follows: proteins identified in all three groups analyzed (7, red); proteins identified in both hamster and mouse vaccinated with a dose of 10⁷ leptospires of the attenuated-vaccine (31, yellow); proteins identified between the group of mice immunized with different doses and the group of mice immunized with a dose of 10⁷ leptospires (2, green); proteins identified between the group of mice immunized with different doses and the group of hamsters immunized with a dose of 10⁷ leptospires (1, blue); proteins identified only in the group of mice immunized with different doses (3, purple); proteins identified only in the group of mice immunized with a dose of 10⁷ leptospires (16, orange); and proteins identified only in the group of hamsters immunized with a dose of 10⁷ leptospires (94, brown). Enrichment analysis of the reactive antigens was performed compared to the whole proteome of *Leptospira,* based on the PSORB localization (FIG. 5B) and COG (FIG. 5D). Statistical results are represented by *(p<0.05), **(p<0.001), and ***(p<0.0001). See Table 5.

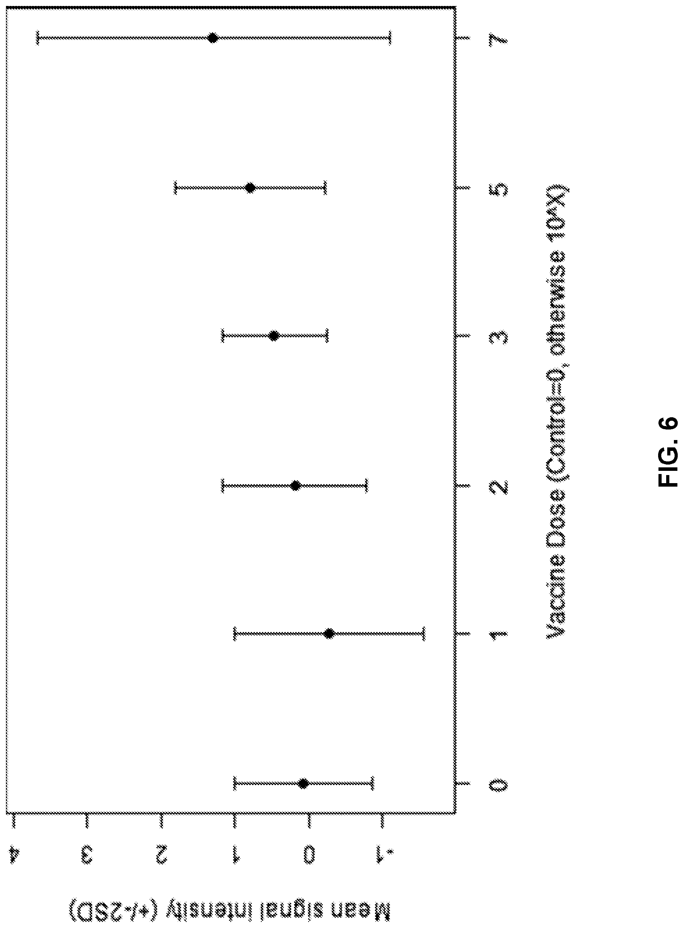

FIG. 6 depicts the mouse dose-response relationship, showing an association between the different doses of the attenuated L1-130 *fcpA⁻* attenuated-vaccine in mice and the mean signal response intensity against all different proteins.
FIG. 7 depicts a heat map of 41 seroreactive proteins recognized by hamsters and mice immunized with attenuated L1-130 *fcpA⁻* attenuated-vaccine. Proteins were selected based on the groups depicted on FIG. 4 and Table 5: present in all three groups of analysis (red), present in both hamster and mice immunized with 10⁷ leptospires (yellow), present in both hamsters immunized with 10⁷ leptospires and mice immunized with a dose range (blue), and present in both mice immunized with 10⁷leptospires and mice immunized with a dose range (green). The proteins are identified by their L. interrogans serovar Copenhageni ORF number and the heat-map shows the signal intensity of antibody response (based on log-fold change) in all animals vaccinated with the *fcpA⁻* mutant used for this analysis (37 hamsters and 34 mice). Right panel shows amino acid sequence identity of respective ORFs among a representative of all pathogenic *Leptospira* species.

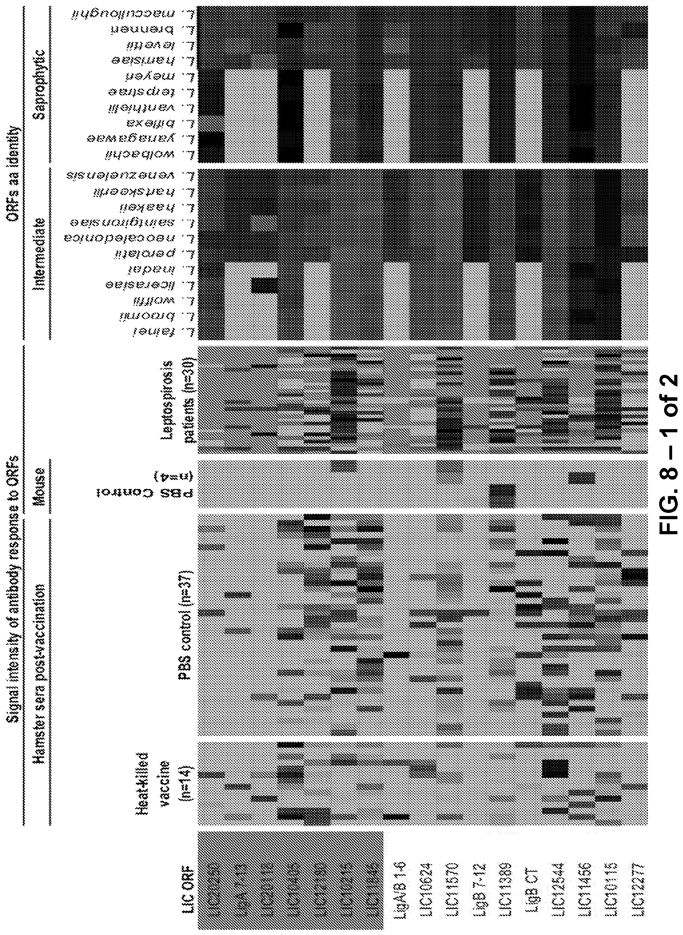

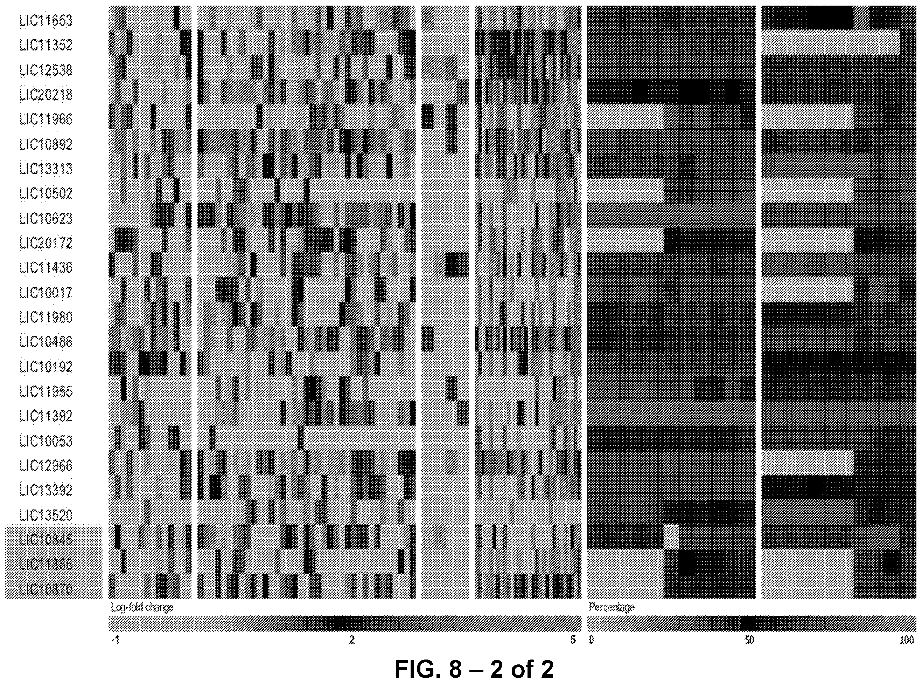

FIG. 8 depicts a complementary heat-map of 41 seroreactive proteins recognized by hamsters and mice immunized with attenuated L1-130 *fcpA⁻*attenuated-vaccine. Proteins were selected based on the groups depicted on FIG. 4 and Table 5: present in all three groups of analysis (red), present in both hamster and mice immunized with 10⁷ leptospires (yellow), present in both hamsters immunized with 10⁷ leptospires and mice immunized with a dose range (blue), and present in both mice immunized with 10⁷ leptospires and mice immunized with a dose range (green). The proteins are identified by their L. interrogans serovar Copenhageni ORF number and the heat-map shows the signal intensity of antibody response (based on log-fold change) in all control animals used for this analysis (14 hamsters vaccinated with heat-killed vaccine, 37 PBS control hamsters, and 4 PBS control mice). The heat-map also shows the result for 30 leptospirosis patients. Right panel shows amino acid sequence identity of respective ORFs among a representative of all intermediate and saprophytic *Leptospira* species.

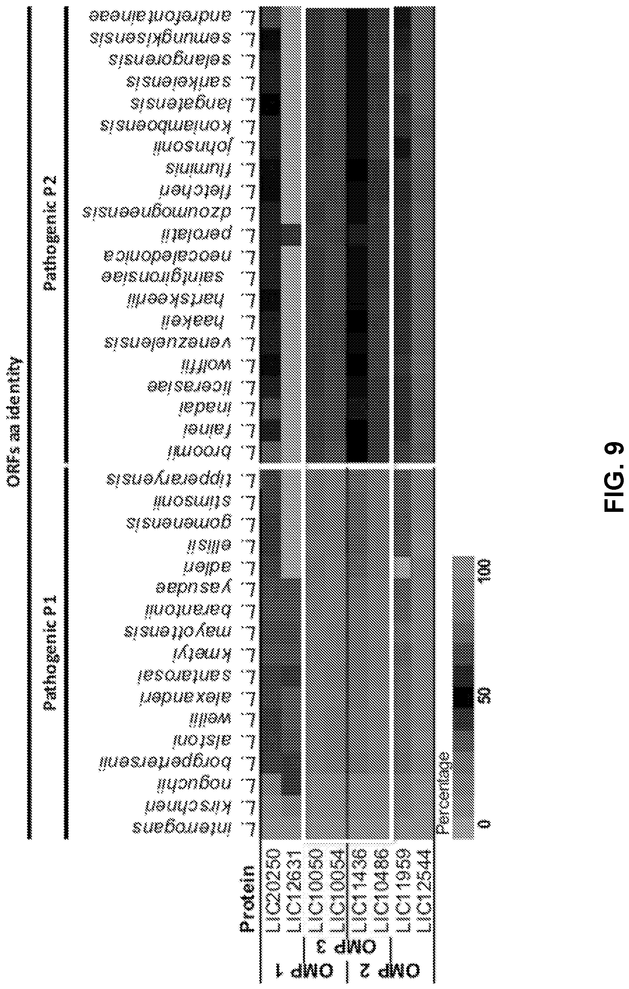

FIG. 9 depicts the 8 targets selected among the list of outer membrane proteins (OMP) identified by the attenuated vaccine: LIC20250 (SEQ ID NO:1), LIC12631 (SEQ ID NO: 2), LIC10050 (SEQ ID NO: 3), LIC10054 (SEQ ID NO: 4), LIC11436 (SEQ ID NO: 5), LIC10486 (SEQ ID NO: 6), LIC11959 (SEQ ID NO: 7), and LIC12544 (SEQ ID NO: 8). Combination "OMP1" comprises LIC20250, LIC12631, LIC10050, and LIC10054; combination "OMP2" comprises LIC11436, LIC10486, LIC11959, and LIC12544; and combination "OMP3" comprises LIC10050, LIC10054, LIC11436, and LIC10486.

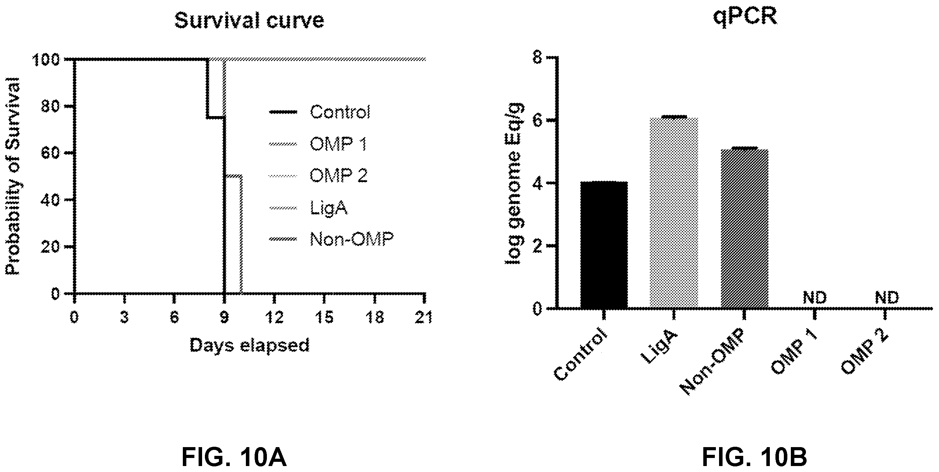

FIGs. 10A-10B depict survival and renal colonization upon vaccination with "OMP1" and "OMP2" in a hamster model. FIG. 10A, survival curve; FIG. 10B, renal colonization determined by qPCR, upon challenge with serovar Copenhageni.

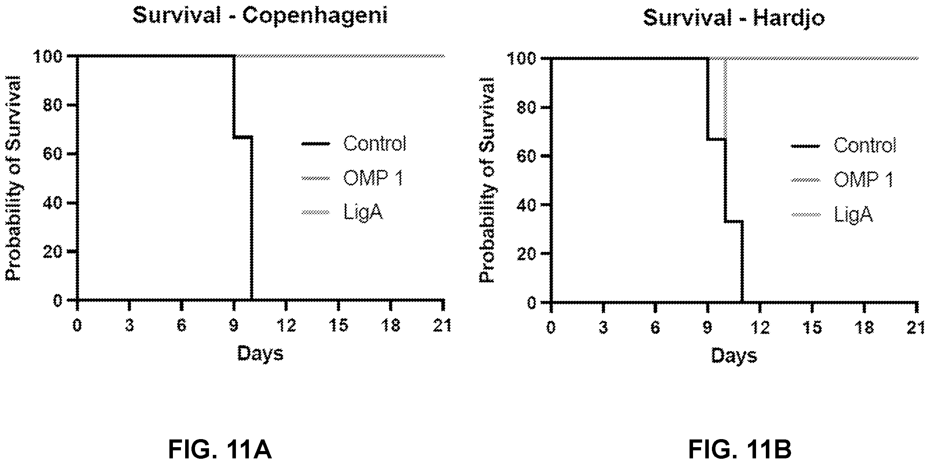

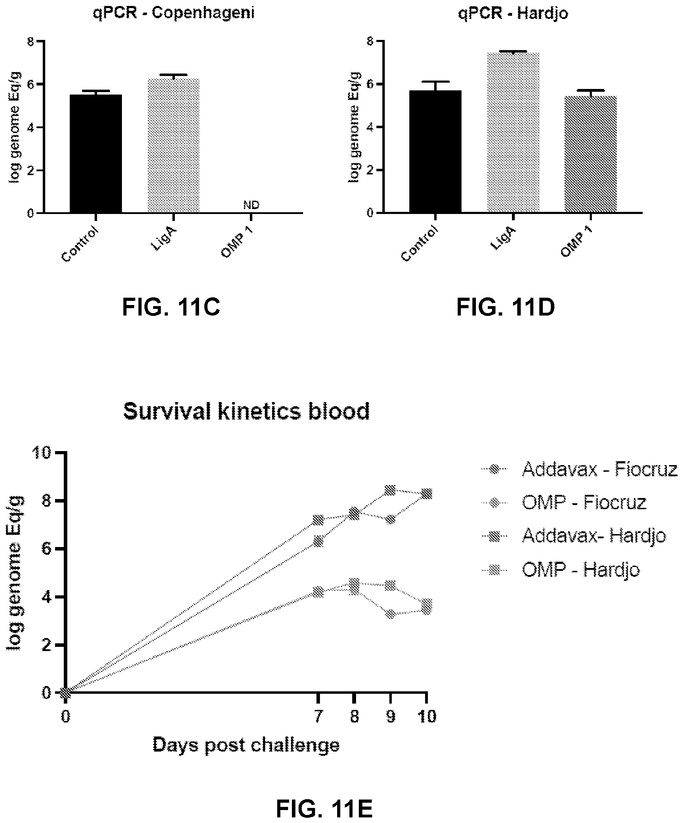

FIGs. 11A-11E depict survival and renal colonization upon vaccination with "OMP1". FIG. 11A, protection against death upon challenge with serovar Copenhageni (homologous); FIG. 11B, cross protection against death upon challenge with serovar Hardjo (heterologous); FIG. 11C, renal colonization in hamsters challenged with serovar Copenhageni; FIG. 11D, renal colonization in hamsters challenged with serovar Hardjo; FIG. 11E, survival kinetics of serovar Copenhageni (Fiocruz) in blood.

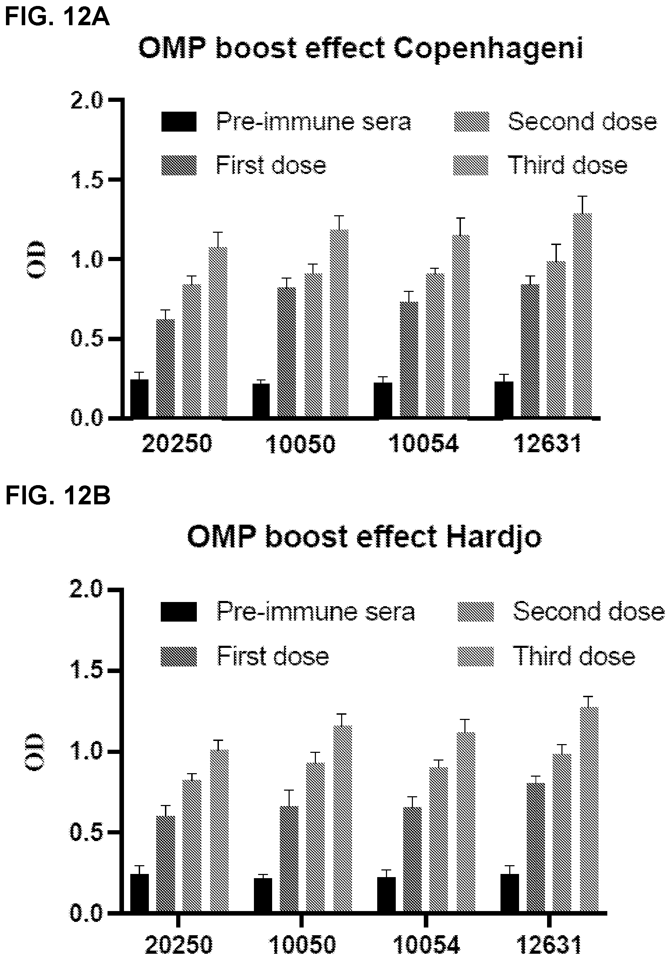

FIGs. 12A-12B depict the immunogenicity of the "OMP1" multi-recombinant construct. FIG. 12A, effect of boosting of hamsters with "OMP1" on antibody production towards serovar Copenhageni; FIG. 12B, effect of boosting with "OMP1" on antibody production against serovar Hardjo.

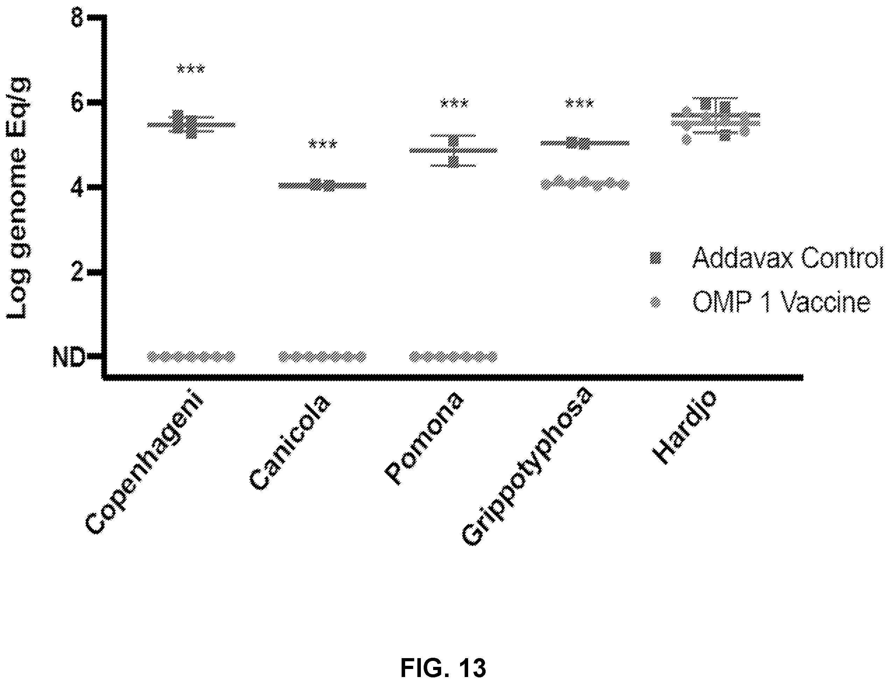

FIG. 13 depicts the cross protection of "OMP1" multi recombinant construct against L. interrogans (serovars Copenhageni, Pomona and Canicola) on renal colonization in hamsters.

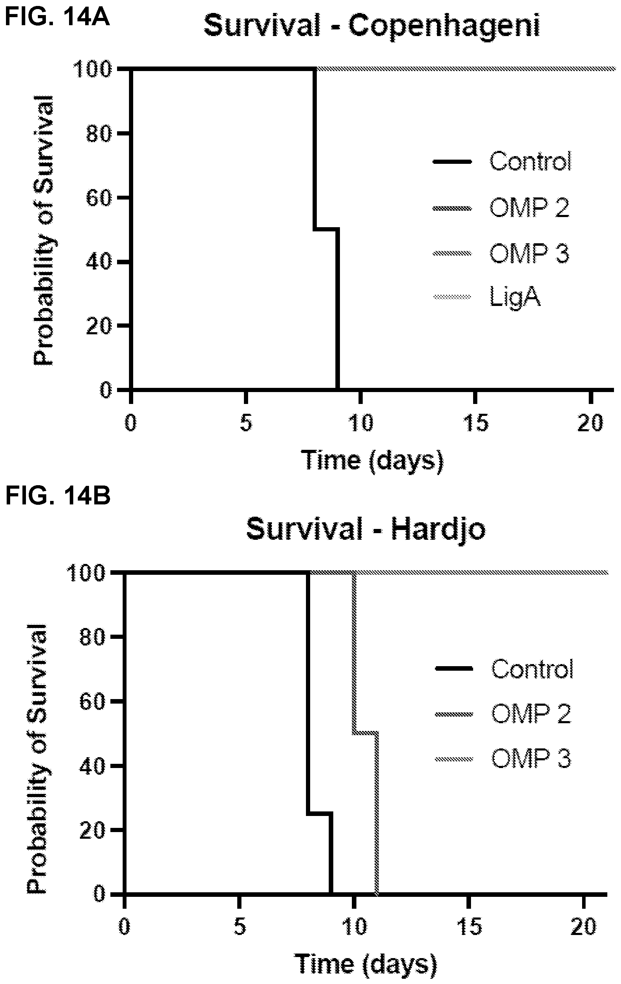

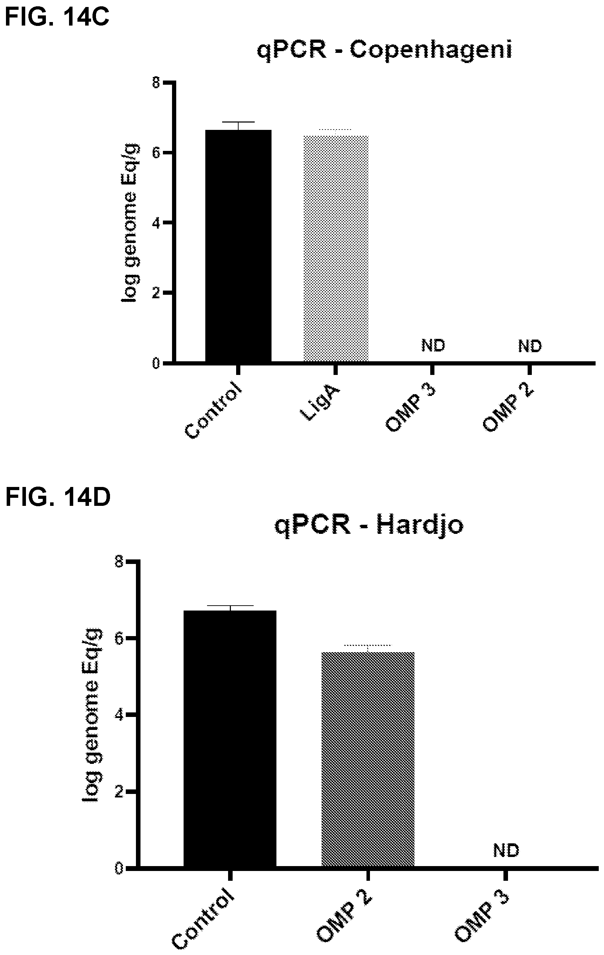

FIGs. 14A-14D show that the composition of the multi recombinant construct influences the ability to induce cross protection against death and renal colonization. FIG. 14A, survival of hamsters immunized with "OMP2" and "OMP3" upon challenge with serovar Copenhageni; FIG. 14B, survival of hamsters immunized with "OMP2" and "OMP3" upon challenge with serovar Hardjo; FIG. 14C, renal colonization in hamsters immunized with "OMP2" and "OMP3" upon challenge with serovar Copenhageni; FIG. 14D, renal colonization in hamsters immunized with "OMP2" and "OMP3" upon challenge with serovar Hardjo.

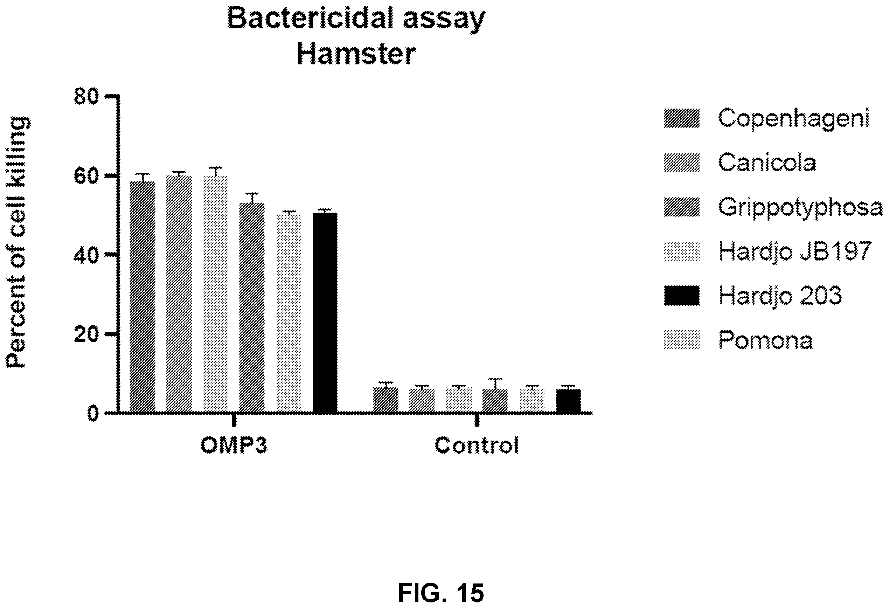

FIG. 15 depicts that anti-"OMP3" sera induces high levels of cross bactericidal activity (50-60%).

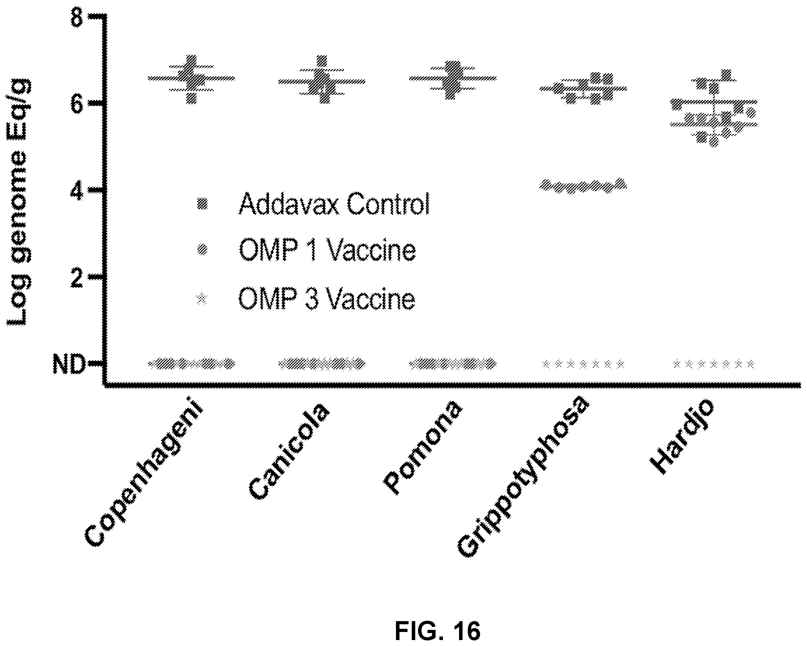

FIG. 16 is a graph showing that "OMP3" elicits sterilizing immunity across species in the hamster model.

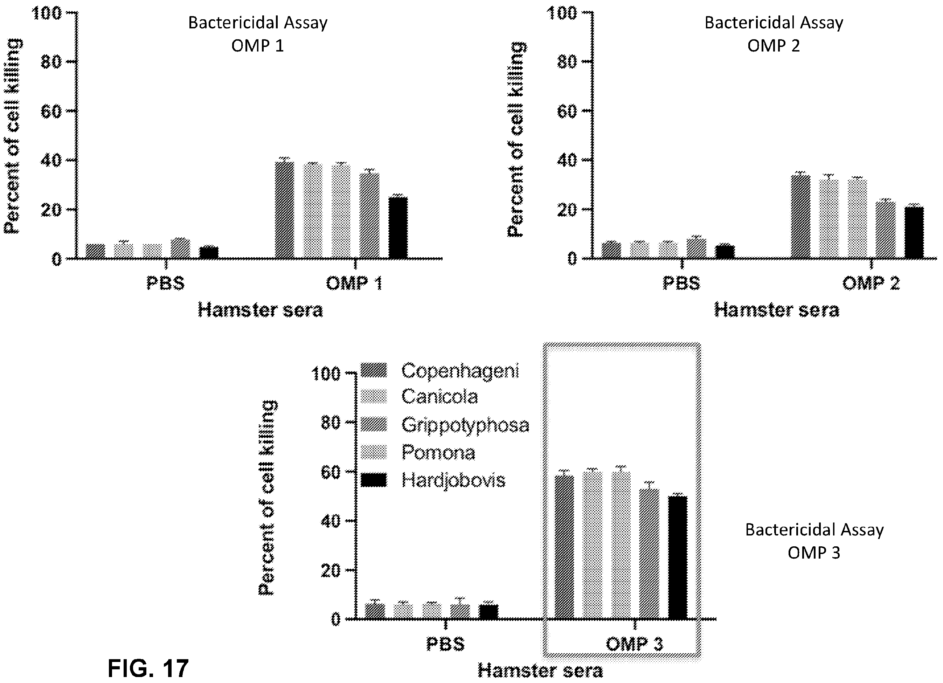

FIG. 17 are graphs showing that sera of animals vaccinated with "OMP3" induces higher levels of cross bactericidal activity compared to sera of animals vaccinated with "OMP1" or "OMP2".

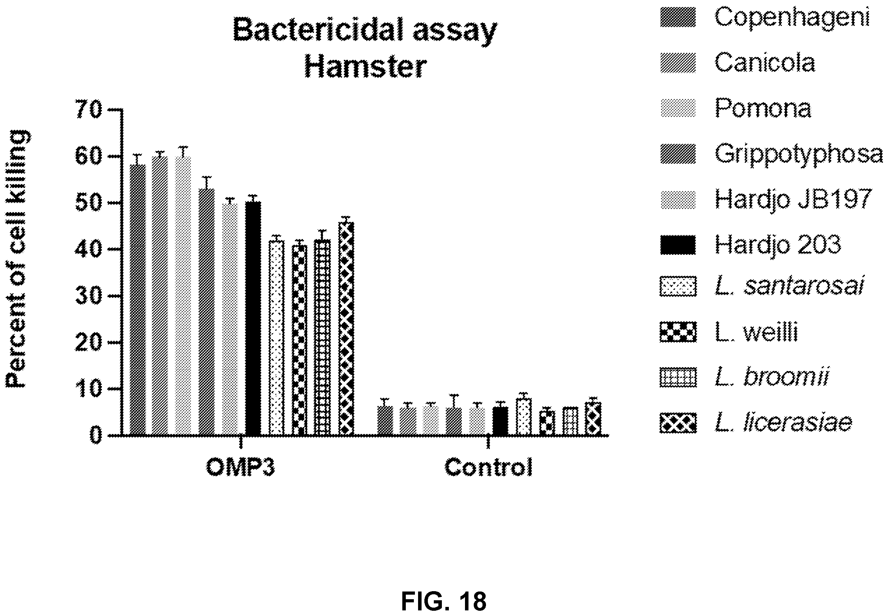

FIG. 18 is a graph showing that sera of animals vaccinated with "OMP3" induces high levels of cross bactericidal activity against P1 and P2 of *Leptospira* spp. strains, for which animal experiments cannot be conducted.

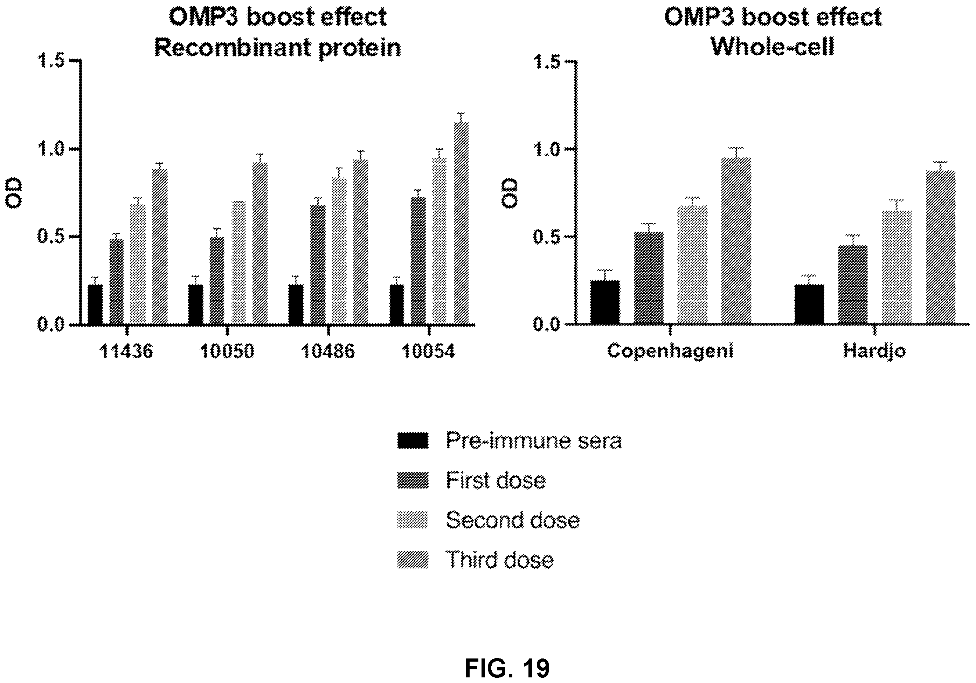

FIG. 19 are graphs showing that the immune response of the "OMP3" construct after each immunization induced a significant boost effect for all proteins and whole-cell leptospires.

### DETAILED DESCRIPTION OF THE INVENTION

The present disclosure relates to compositions and methods for inducing an immune response against *Leptospira* for the prevention and treatment of leptospirosis. In one aspect, the disclosure provides a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8. In certain embodiments, the composition consists essentially of SEQ ID NO: 3, SEQ ID NO: 4, SEQ ID NO: 5, and SEQ ID NO: 6, collectively referred to herein as "OMP3". In some embodiments, the composition consists essentially of SEQ ID NO: 3, SEQ ID NO: 4, SEQ ID NO: 5, SEQ ID NO: 6, and an adjuvant.

### Definitions

Unless defined otherwise, all technical and scientific terms used herein have the same meaning as commonly understood by one of ordinary skill in the art to which the invention pertains. Although any methods and materials similar or equivalent to those described herein may be used in the practice of an/or for the testing of the present invention, the preferred materials and methods are described herein. In describing and claiming the present invention, the following terminology will be used according to how it is defined, where a definition is provided.

It is also to be understood that the terminology used herein is for the purpose of describing particular embodiments only, and is not intended to be limiting.

It is also to be understood that the methods described in this disclosure are not limited to particular methods and experimental conditions disclosed herein as such methods and conditions may vary.

Furthermore, the experiments described herein, unless otherwise indicated, use conventional molecular and cellular biological and immunological techniques within the skill of the art. Such techniques are well known to the skilled worker, and are explained fully in the literature. See, *e.g.,* Ausubel, et al., ed., Current Protocols in Molecular Biology, John Wiley & Sons, Inc., NY, N.Y. (1987-2008), including all supplements, Molecular Cloning: A Laboratory Manual (Fourth Edition) by MR Green and J. Sambrook, and Harlow et al., Antibodies: A Laboratory Manual, Chapter 14, Cold Spring Harbor Laboratory, Cold Spring Harbor (2013, 2nd edition).

Unless otherwise defined, scientific and technical terms used herein have the meanings that are commonly understood by those of ordinary skill in the art. In the event of any latent ambiguity, definitions provided herein take precedent over any dictionary or extrinsic definition. Unless otherwise required by context, singular terms shall include pluralities and plural terms shall include the singular. The use of "or" means "and/or" unless stated otherwise. The use of the term "including," as well as other forms, such as "includes" and "included," is not limiting.

Generally, nomenclature used in connection with cell and tissue culture, molecular biology, immunology, microbiology, genetics and protein and nucleic acid chemistry and hybridization described herein is well-known and commonly used in the art. The methods and techniques provided herein are generally performed according to conventional methods well known in the art and as described in various general and more specific references that are cited and discussed throughout the present specification unless otherwise indicated. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well-known and commonly used in the art. Standard techniques are used for chemical syntheses, chemical analyses, pharmaceutical preparation, formulation, and delivery, and treatment of patients.

Compounds of the present disclosure may contain one or more asymmetric centers and may thus give rise to enantiomers, diastereomers, and other stereoisomeric forms. The present disclosure is meant to encompass all such possible forms, as well as their racemic and resolved forms and mixtures thereof.
So that the disclosure may be more readily understood, select terms are defined below.

As used herein, the articles "a" and "an" are used to refer to one or to more than one *(i.e.,* to at least one) of the grammatical object of the article. By way of example, "an element" means one element or more than one element.

As used herein when referring to a measurable value such as an amount, a temporal duration, and the like, the term "about" is meant to encompass variations of ±20% or within 10%, 9%, 8%, 7%, 6%, 5%, 4%, 3%, 2%, 1%, 0.5%, 0.1%, 0.05%, or 0.01% of the specified value, as such variations are appropriate to perform the disclosed methods. Unless otherwise clear from context, all numerical values provided herein are modified by the term about.

The term "antibody" or "Ab" as used herein, refers to a protein, or polypeptide sequence derived from an immunoglobulin molecule, which specifically binds to a specific epitope on an antigen. Antibodies can be intact immunoglobulins derived from natural sources or from recombinant sources and can be immunoreactive portions of intact immunoglobulins. The antibodies useful in the present invention may exist in a variety of forms including, for example, polyclonal antibodies, monoclonal antibodies, intracellular antibodies ("intrabodies"), Fv, Fab and F(ab)2, as well as single chain antibodies (scFv) and humanized antibodies (Harlow et al., 1998, Using Antibodies: A Laboratory Manual, Cold Spring Harbor Laboratory Press, NY; Harlow et al., 1989, Antibodies: A Laboratory Manual, Cold Spring Harbor, New York; Houston et al., 1988, Proc. Natl. Acad. Sci. USA 85:5879-5883; Bird et al., 1988, Science 242:423-426). An antibody may be derived from natural sources or from recombinant sources. Antibodies are typically tetramers of immunoglobulin molecules.

The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full-length nucleotide sequence of a gene. It is readily apparent that the present invention includes, but is not limited to, the use of partial nucleotide sequences of more than one gene and that these nucleotide sequences are arranged in various combinations to elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

"Heterologous antigens" used herein to refer to an antigen that is not endogenous to the organism comprising or expressing an antigen. The term "Heterologous protein" as used herein refers to a protein that elicits a beneficial immune response in a subject (i.e. mammal), irrespective of its source.

The term "specifically binds", "selectively binds" or "binding specificity" refers to the ability of the humanized antibodies or binding compounds of the invention to bind to a target epitope with a greater affinity than that which results when bound to a non-target epitope. In certain embodiments, specific binding refers to binding to a target with an affinity that is at least 10, 50, 100, 250, 500, or 1000 times greater than the affinity for a non-target epitope.

As used herein, by "combination therapy" is meant that a first agent is administered in conjunction with another agent. "In combination with" or "In conjunction with" refers to administration of one treatment modality in addition to another treatment modality. As such, "in combination with" refers to administration of one treatment modality before, during, or after delivery of the other treatment modality to the individual. Such combinations are considered to be part of a single treatment regimen or regime.

"Humoral immunity" or "humoral immune response" both refer to B-cell mediated immunity and are mediated by highly specific antibodies, produced and secreted by B-lymphocytes (B-cells).

"Incorporated into" or "encapsulated in" refers to an antigenic protein or peptide that is within a delivery vehicle, such as microparticles, bacterial ghosts, attenuated bacteria, virus like particles, attenuated viruses, ISCOMs, liposomes and preferably virosomes.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that may comprise a protein or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "isolated" as used herein in relation to polypeptides, as in "isolated polypeptides," refers to polypeptides, which are isolated from other cellular proteins and is meant to encompass both purified and recombinant polypeptides. The term "isolated polypeptide(s)" thus refers to a polypeptide/polypeptides that is/are substantially free of cellular material, viral material, or culture medium when produced by recombinant DNA techniques, or chemical precursors or other chemicals when chemically synthesized.

"Identity" as used herein refers to the subunit sequence identity between two polymeric molecules, particularly between two amino acid molecules, such as, between two polypeptide molecules. When two amino acid sequences have the same residues at the same positions, e.g., if a position in each of two polypeptide molecules is occupied by an Arginine, then they are identical at that position. The identity or extent to which two amino acid sequences have the same residues at the same positions in an alignment is often expressed as a percentage. The identity between two amino acid sequences is a direct function of the number of matching or identical positions; e.g., if half (e.g., five positions in a polymer ten amino acids in length) of the positions in two sequences are identical, the two sequences are 50% identical; if 90% of the positions (e.g., 9 of 10), are matched or identical, the two amino acids sequences are 90% identical.

A "subject" or "patient," as used therein, may be a human or non-human mammal. Non-human mammals include, for example, livestock and pets, such as ovine, bovine, porcine, canine, feline, and murine mammals. Preferably, the subject is human. In some embodiments, the subject is a domestic pet or livestock. In some embodiments, the subject is a mouse. In some embodiments, the subject is a hamster.

The term "biological sample" refers to a sample obtained from an organism or from components (e.g., cells) of an organism. The sample may be of any biological tissue or fluid. Frequently the sample will be a "clinical sample" which is a sample derived from a patient. Such samples include, but are not limited to, bone marrow, cardiac tissue, sputum, blood, lymphatic fluid, blood cells (e.g., white cells), tissue or fine needle biopsy samples, urine, peritoneal fluid, and pleural fluid, or cells therefrom. Biological samples may also include sections of tissues such as frozen sections taken for histological purposes.

"Vaccination" refers to the process of inoculating a subject with at least one antigen to elicit an immune response in the subject, that helps to prevent or treat the disease or disorder the antigen is connected with. The term "immunization" is used interchangeably herein with vaccination.

By *"Leptospira* bacterium" or *"Leptospira* bacteria" is meant a spirochete bacterium or bacteria. *Leptospira* bacteria are very thin, tightly coiled, obligate aerobic spirochetes characterized by a unique flexuous type of motility. *Leptospira* bacterium is a gram-negative spirochete with internal flagella. The genus is divided into 64 species: the pathogenic leptospires represented as P1 and P2 and the free-living leptospires represented as S1 and S2. Serotypes of P1 and P2 are the agents of leptospirosis, a zoonotic disease.

By "pathogen" is meant an infectious agent, such as *Leptospira* bacteria, capable of causing infection, producing toxins, and/or causing disease in a host.

By "Flagellar-coiling protein A" or "FcpA" is meant an abundant protein from *Leptospira* spp., exposed on the periplasmic flagella surface. The gene encoding FcpA is highly conserved among *Leptospira* and is not found in other bacteria. FcpA(-) mutants, obtained from clinical isolates or by allelic exchange, are not able to produce translational motility and do not cause disease in the standard hamster model of leptospirosis.

As applied to the nucleic acid or protein, "homologous" as used herein refers to a sequence that has about 50% sequence identity. More preferably, the homologous sequence has about 75% sequence identity, even more preferably, has at least about 80%, 85%, 90%, 91%, 92%, 93%, 94%, 95%, 96%, 97%, 98%, 99% sequence identity.

The term " immunogenicity" as used herein, refers to the innate ability of an antigen or organism to elicit an immune response in an animal when the antigen or organism is administered to the animal. Thus, "enhancing the immunogenicity" refers to increasing the ability of an antigen or organism to elicit an immune response in an animal when the antigen or organism is administered to an animal. The increased ability of an antigen or organism to elicit an immune response can be measured by, among other things, a greater number of antibodies that bind to an antigen or organism, a greater diversity of antibodies to an antigen or organism, a greater number of T-cells specific for an antigen or organism, a greater cytotoxic or helper T-cell response to an antigen or organism, a greater expression of cytokines in response to an antigen, and the like.

A "fragment" of a polypeptide means at least about five to about 25 sequential amino acids of the polynucleotide. It is understood that a portion of a polypeptide may include every amino acid residue of the polypeptide. An "immunogenic fragment" is a fragment capable of inducing an immune response in a subject.

"Adjuvant" refers to a substance that is capable of potentiating the immunogenicity of an antigen. Adjuvants can be one substance or a mixture of substances and function by acting directly on the immune system or by providing a slow release of an antigen. Examples of adjuvants are aluminium salts, polyanions, bacterial glycopeptides and slow release agents as Freund's incomplete or AddaVax^{™}, which is an oil-in-water nano-emulsion adjuvant.

"Delivery vehicle" refers to a composition that helps to target the antigen to specific cells and to facilitate the effective recognition of an antigen by the immune system. The best-known delivery vehicles are liposomes, virosomes, microparticles including microspheres and nanospheres, polymers, bacterial ghosts, bacterial polysaccharides, attenuated bacteria, virus like particles, attenuated viruses and ISCOMS.By "attenuated" is meant the bacterium has a decreased virulence with respect to a wild-type bacterium. In particular, a bacterium has an attenuated virulence of about 10, 20, 30, 40, 50, 60, 70, 80% or more decrease in virulence as compared to a wild-type bacterium.

As used herein, the term "pharmaceutical composition" refers to a mixture of at least one compound useful within the invention with other chemical components, such as carriers, stabilizers, diluents, adjuvants, dispersing agents, suspending agents, thickening agents, and/or excipients. The pharmaceutical composition facilitates administration of the compound to an organism. Multiple techniques of administering a compound exist in the art including, but not limited to: intravenous, oral, aerosol, parenteral, ophthalmic, pulmonary and topical administration.

The language "pharmaceutically acceptable carrier" includes a pharmaceutically acceptable salt, pharmaceutically acceptable material, composition or carrier, such as a liquid or solid filler, diluent, excipient, solvent or encapsulating material, involved in carrying or transporting a compound(s) of the present invention within or to the subject such that it may perform its intended function. Typically, such compounds are carried or transported from one organ, or portion of the body, to another organ, or portion of the body. Each salt or carrier must be "acceptable" in the sense of being compatible with the other ingredients of the formulation, and not injurious to the subject. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; diluent; granulating agent; lubricant; binder; disintegrating agent; wetting agent; emulsifier; coloring agent; release agent; coating agent; sweetening agent; flavoring agent; perfuming agent; preservative; antioxidant; plasticizer; gelling agent; thickener; hardener; setting agent; suspending agent; surfactant; humectant; carrier; stabilizer; and other non-toxic compatible substances employed in pharmaceutical formulations, or any combination thereof. As used herein, "pharmaceutically acceptable carrier" also includes any and all coatings, antibacterial and antifungal agents, and absorption delaying agents, and the like that are compatible with the activity of the compound, and are physiologically acceptable to the subject. Supplementary active compounds may also be incorporated into the compositions.

By "effective amount" is meant the amount required to reduce or improve at least one symptom of a disorder, condition or disease relative to an untreated patient. The effective amount used for therapeutic treatment of a condition or disease or stimulating an immune response, varies depending upon the manner of the specific disorder, condition or disease, extent of the disorder, condition or disease, and administration of the cells, as well as the age, body weight, and general health of the subject.

By the term "vaccine" as used herein, is meant a composition, a bacterium, a protein, or a nucleic acid of the invention, which serves to protect an animal against a *Leptospira* bacterial disease and/or to treat an animal already infected with *Leptospira* bacteria compared with an otherwise identical animal to which the vaccine is not administered or compared with the animal prior to the administration of the vaccine.

By "virulence" is meant a degree of pathogenicity of a given pathogen or the ability of an organism to cause disease in another organism. Virulence refers to an ability to invade a host organism, cause disease, evade an immune response, and produce toxins.

By "bacterial virulence" is meant a degree of pathogenicity of bacteria, such as *Leptospira* bacteria. Bacterial virulence includes causing infection or disease in a host, producing agents that cause or enhance disease in a host, producing agents that cause or enhance disease spread to another host, and causing infection or disease in another host.

By "virulent" or "pathogenic" is meant a capability of a bacterium to cause a severe disease.

By "non-pathogenic" is meant an inability to cause disease.

By "wildtype" is meant a non-mutated version of a gene, allele, genotype, polypeptide, or phenotype, or a fragment of any of these. It may occur in nature or produced recombinantly.

By "infection" is meant a bacterial colonization of the host. Infection of a host can occur by entry of the bacterium or bacteria through a break in barrier epithelial surfaces, such as unhealed breaks in the skin, the eyes, or with the mucous membranes.

By "infectious disease" is meant a disease or condition in a subject caused by a pathogen that is capable of being transmitted or communicated to a non-infected subject. Non-limiting examples of infectious diseases include bacterial infections, viral infections, fungal infections, and the like.

In this disclosure, "comprises," "comprising," "containing" and "having" and the like can have the meaning ascribed to them in U.S. Patent law and can mean "includes," "including," and the like; "consisting essentially of" or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments. In some embodiments, the term "consisting essentially of" refers to a composition, whose only active ingredient is the indicated active ingredient(s) (e.g., the indicated peptides), however, other compounds may be included which are for stabilizing, preserving, etc. the formulation, but are not involved directly in the therapeutic effect of the indicated active ingredient. Use of the transitional phrase "consisting essentially of" means that the scope of a claim is to be interpreted to encompass the specified materials or steps recited in the claim, and those that do not materially affect the basic and novel characteristic(s) of the claimed invention. Thus, the term "consisting essentially of" when used in a claim of this invention is not intended to be interpreted to be equivalent to "comprising."

As used herein, the terms "control," or " reference" are used interchangeably and refer to a value that is used as a standard of comparison. A control subject is for example a subject that is immunized with adjuvant only, or that is immunized with peptides or proteins that are not expected to elicit an immune response.

As used herein, the terms "eliciting an immune response" or "immunizing" refer to the process of generating a B cell and/or a T cell response against a heterologous protein.

As used herein, the terms "treat," treating," "ameliorating," "treatment," and the like refer to reducing or improving an infectious disease or condition and/or one or more symptoms associated therewith. It will be appreciated that, although not precluded, treating an infectious disease or condition and/or one or more symptoms associated therewith does not require that the disorder, condition, disease or symptoms associated therewith be completely ameliorated or eliminated. It means that the clinical signs and/or the symptoms associated with a disease are lessened as a result of the actions performed. The signs or symptoms to be monitored will be well known to the skilled clinician.

"Prevention" refers to the use of a pharmaceutical compositions for the vaccination against a disorder.

By "immune response" is meant the actions taken by a host to defend itself from pathogens or abnormalities. The immune response includes innate (natural) immune responses and adaptive (acquired) immune responses. Innate responses are antigen non-specific. Adaptive immune responses are antigen specific. An immune response in an organism provides protection to the organism against *Leptospira* bacterial infections when compared with an otherwise identical subject to which the composition or cells were not administered or to the human prior to such administration.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

### Composition

Without being limited by theory, the invention is based in part on the discovery that inoculation with certain leptospira peptides can provide immunity against leptospirosis generally, regardless of serovar. The disclosure provides a universal vaccine against leptospirosis using a multi-recombinant protein construct. In some embodiments, the multi-recombinant protein construct comprises an effective amount of 2, 3, 4. 5, 6, 7, or 8 polypeptides, or fragments thereof, selected from:
SEQ ID NO: 1 (LIC20250; Uncharacterized OmpA-like protein);
SEQ ID NO: 2 (LIC12631; Hemolysin);
SEQ ID NO: 3 (LIC10050; Uncharacterized OmpA-like protein);
SEQ ID NO: 4 (LIC10054; Uncharacterized lipoprotein);
SEQ ID NO: 5 (LIC11436; Uncharacterized FecR domain-containing protein);
SEQ ID NO: 6 (LIC10486; Uncharacterized protein);
SEQ ID NO: 7 (LIC11959; Uncharacterized protein); and
SEQ ID NO: 8 (LIC12544; Uncharacterized DNA binding protein).

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 1.

MAKKENYYITIKGRKYDRKLIQLAEEFTSGKRDGKISINDAKRLLKIVKDNNA
YTDIEKHTIEYIRENYKFTEKSDEWFRSEIRKWAAKKVQEAKKKSDVESILVD
DSEAPEINFPSSWGEDKTEVVEITQTSKIDWRENSNFSSATSHSKKNKKIIPTLI
FLSGFLILVGLVYFFRTLFYKEDLEQVVKTNSEIVSNSKEKQSDVSIEKAESTK
EVRKKNVRSKKEESEIPKNALTILKPQTGKKLESKSLFSSLTNQNSTEEFSSNP
QFREIESNVIRFEKNSIQIHKESRPSLNRLARWMKQDSSIRVKVIGHTSLEGSED
ANQKVSLLRAQTVRNYIAGNGISKDRFEIIPKGASVPIGDNSKEEGKEMNRRV
ELRIYN (SEQ ID NO: 1)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 2.

MINKITKPKLLIGYYLLLFSLIRCLPEKESSYKDLFTSLLFLPNQTNSNQVNSVS
INNDPANPNPVNPASANNNQVNAVPENDNPANLNPVNPASANSNQVNAAPE
NGSSADPNPANLASGNNNQVNAVPANNYFTKEDSSNNIPKKVNSKNVEIKVL
SHNVFMLPTNLPRWGNLGHDERAKRISKSDYVKNQDVIVFEEAFDTSARKIL
LDNLREEYPYQTDVVGRTKKNWDASLGNFRSYSLVNGGVVILSKWPIEEKIQ
YIFNDSGCGTDWFANKGFVYVKINKEGKKFHVIGTHAQSQDQNCSNLGIPNR
ANQFDDIRNFIYSKNIPKDETVLIVGDLNVIKESNEYYDMISRLNVNEPRYVGV
PFTWDAKTNEIAAYYYENEEPVYLDYIFVSKSHAQPPVWQNLAYDPVSKQT

WTVSGYTSDEFSDHYPIYGFVYADPSTPTKSGHKKKYDQVSFQSAANGKYIQ
ADPNRKNGWLKADAVIETDFTKFNLLQEGNLNPSCIKNGLVRIESSRFLNYFW
NWWLGGGSGNYGYYSKFNDASNQLEIINLSDGCLENGSKIVFKDYDTYSRNH
YYLTVWDKGNWNEHLYLWKDSISQREIFYLKLNSTPVRNWSADLIYR (SEQ
ID NO: 2)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 3.

MRPFSKLIFILAFCIFLPVFSQPLPDLPEKQFGQPLNTQNDEYNPIVSPDGRYIVF
QSNRPGGEGGMDIWISENIRFLDKEIPAEWTKPVNMNQNIWEELKRPPAAGV
RKPNLFNSNAFEGGISILFDSNNAPSEIYFTSTINLAVGRSGFEGLNIYRTIKDK
KTGRWTDPEHLSEINSNFNDKMPAISPDGNFLIFSSDRPGGYGDFDLWISVRN
PKNGSWSQPKNLGSPLNSSESEILPFIHQDGEQLYFSSNREDERKKFKIFRIFLK
YKSALDNMLEDEEETEEIPTTKPTEILIPKIDQSSLLLLPKPFNTDKWEGFDNEG
INFDKDGIWAYISSNRSGGEGQFDIFRFQVPESIRNSYTLNFKGLVLDGSEKTM
IGLDSTLKIYDGTKPANVITSKRIGGDLSKGKPSNFATTLQTGKVYKIEISSPGF
HPQEDILDLRGNIGKNRKVYRTYVLLPIQVGEGKTEETKIEQPIENQKPNSAAL
KVIVADASTKQIIPDAKVTLFTPMNRKGESLVQDADKKSFLIKKLPDNDFELF
ATASKYISESINIIQKNISKNGTVTIYLKAESDVDPVYNLRVYFEFNKTKITEEN
KKLLDPLVGYLLKNASDKIEIGGHTDNVASKEYNTRLSAKRARNVYEYLLSK
GIPEKRMRIRAYWYSQPDADNETETGRAKNRRVGFRKL (SEQ ID NO: 3)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 4.

MVFSRSTDLFKGINRTEETMKQIAILTALIIFTSCASVESKRSVSASGDPSEIFFE
KEIIPMDSNSNFVSQKPARRSFEEELSVEKYAKAQPPEKTNSSGDFDEVGMSS
WYGAKFHGKPTASGEKFDKTKLTAAHPTLPLGSIIRVQNLENQKEVIVRVND
RGPFVKDRIIDLSEKAADTLDFKDVGIAKVGIKVVKRGGAANEESEDLENSDD
EEALLEDGKPEKLNPQKSDYQNKPIAGGKYIKGAPKGYTVQVGVFREQSRAE
SYKSNLGQEYGEKTFLFTRDGLFVIQLGDFASRTEAESLKSKLKNDGIDCFIPK
K (SEQ ID NO: 4)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 5.

MKYLTEEKYVVTVLTGLILFFSILLYFHITSGHKKGSNPEIGKIIFKNRKAQRK
YDSEVLWEEIETEMKVRNRDTVRTDDGAEAVLVLNDGTEIKLDQKSMIFLDF
SDKNLSIDFAYGSVSANKESGTELQIKSGETTVEVGKGDLKLSKTEDQALNLE
VSKGNAKVKSGNQESNVSNNQAIELKNGKSEIRSLSISLNSPTERKFFQTSSNS
FPISFSWNKAESAKEYTLEISNHPSFSKNVIRTKSNGTSLNRSLEKGTHYWRVT
AINPGTGTPEFSETRSLIVLGELKSSLFTPAKSEEFKFTSNVPSIVFQWTPVDFT
NNYTFELAKDKEFKEILINQEVQGTLYRWDKTKEGKYFARVTPKPSLNDLKA
IPSDPVSFNVRKLEKPEPPVLKKPSDQEEISLRKFSKEGNLFVWSGSADFSEYT
LEIANDSEFKNILFNKKTNSSSLISSPISNAGTYFWRVKGTLKEGDPIFTTVRQF
KVQSLENLELLFPANEQELGHPANHKLTFRWQRPEPSGVYKLEVSKNSEFSG
EVIRENFRSSFGTVSIPSAGEYFWKVSLLGSNGENLISSKTQKFKTSDSTPFLSQ
SSPATEETIDISNRESIDFRWETEGNTESVILEILEKKAGKNKSIFKKEIKGDSYS
FKDFGILEEGKFTWRLSAKYKDKTGIQKFTIPVSRNFEIKLNKTIRPPEVLSPKE
IYVE (SEQ ID NO: 5)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 6.

MEPSNQVNKLQEQANLMNLALESVLTEEQAVELIQGKIRDAYLLKIRIDIENR
SGAVVALVTKYKNEVIEIFSLFSNSSLIRKIRSFQDSAAFALDMVEAAKSEPYD
PGLSDSIGRIVYSKLTKAVLESSYANWDKNDASSLVNILENQIKTSLKVNIVRI
QADVEFISSLKFRAKNVFTGIIPTVNRPVEEPSISQVPESQEKTPVQRQIDQFRK
PFGRVILSKTVLAPVGGVDFDELTEGDRLYFQLPTGSMDEKAMAKTLGGIDE
DGNVRNVVGEFIGIAAGKGEYHIFAKGPSGVLLQAFEERPVRLAKVKTKTSSS
ASTTKTETSSGGGSLGIIIVAGVVLVVGLLVFLIMK (SEQ ID NO: 6)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 7.

LLRFLNIQKILWINFLFLYISSLSVFAQEIHRAASTYRSSISLSEPRISDIKEALSSE
SPNFPNSLKLFFQELKGNYAIFYDWNGETVYYKYRINKFDKSKLKQVRKLSE
GAAYEVNGLWEGLIVFQVSTVPLFKKASEISLEEKKEKSSIPVFDLVEFKELSL
DEILY (SEQ ID NO: 7)

In some embodiments, the composition for stimulating the immune response comprises a polypeptide having at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% sequence identity to SEQ ID NO: 8.

LNQKRVGQILREAREEKKLSVKDVARETNISVKYILALETEDYSQFPGETFTM
GFLKNYGGYLKLDTGQLINLYRGEKIEESQAPLEELTRPTASYYTKINVDKNK
IFTIASVLIILISAYLIIDSFIGPSSDEDSVEESGKKLDIPENIDFLSRSIPESRSESFI
LTPDKGVSFSVSNQQCKLFIDSVEKGGAVNTAVLAFNVYPELTVYKFRLSEG
QEKILSYTIPEISSLRRNVRIISQAVTENSAKVLVTLSDEEQKQENNNSVDNTKT
LGDVPIQVTLFFNKPSYAEFIIDGQMGFRGLVQTGETRSLEAKDRLELKVGDG
SAVEMIQNGKPKITLGRPGKLVKKIFVKTQNPYDSTQSIIKELGE (SEQ ID NO:
8)

In some embodiments, the composition to induce an immune response comprises the proteins of SEQ ID NO: 1, SEQ ID NO: 2, SEQ ID NO: 3, and SEQ ID NO: 4, and are collectively referred to as "OMP1". In some embodiments, the composition to induce an immune response comprises the proteins of SEQ ID NO: 5, SEQ ID NO: 6, SEQ ID NO: 7, and SEQ ID NO:8, and are collectively referred to as "OMP2". In some embodiments, the composition to induce an immune response comprises the proteins of SEQ ID NO: 3, SEQ ID NO: 4, SEQ ID NO: 5, and SEQ ID NO: 6, and are collectively referred to as "OMP3".

In some embodiments, the composition comprises at least four immunogenic fragments from any of SEQ ID NO: 1 to SEQ ID NO: 8. Immunogenic domains in a protein can be identified with suitable software, including, but not limited to AbDesigner. By using such software, or comparable software, features of a protein can be identified with immunogenic properties based on prediction models. The prediction of immunogenic domains in a protein is based on multiple protein factors including hydrophobicity, secondary structure, uniqueness, conservation among species, and the presence or absence of post-translational modifications. Immunogenic peptides typically range from about 10 to about 25 amino acids. In one embodiment, a composition for inducing an immune response comprises one or more immunogenic fragments from any of SEQ ID NO: 1 to SEQ ID NO: 8.

The invention also includes a composition of proteins, or immunogenic fragments thereof, that further includes a pharmaceutically acceptable carrier. In another embodiment, the composition further includes an adjuvant, such as an oil-in-water emulsion, a saponin, a cholesterol, a phospholipid, a CpG, a polysaccharide, variants thereof, and any combination thereof.

In one aspect the invention provides a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8. As used herein, the term "leptospira peptide" refers to peptides found in *Leptospira* bacterium, e.g., the leptospira peptides disclosed herein. In this aspect, each leptospira peptide has 80% or greater sequence identity to one of SEQ ID NOs. 1-8.

In certain embodiments, the composition comprises a plurality of leptospira peptides, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4. In some embodiments, the composition consists essentially of SEQ ID NO: 1, SEQ ID NO: 2, SEQ ID NO: 3, and SEQ ID NO: 4. In some embodiments, the composition consists essentially of SEQ ID NO: 1, SEQ ID NO: 2, SEQ ID NO: 3, SEQ ID NO: 4, and an adjuvant.

In certain embodiments, the composition comprises a plurality of leptospira peptides, wherein the plurality of leptospira peptides comprises a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8. In some embodiments, the composition consists essentially of SEQ ID NO: 5, SEQ ID NO: 6, SEQ ID NO: 7, and SEQ ID NO: 8. In some embodiments, the composition consists essentially of SEQ ID NO: 5, SEQ ID NO: 6, SEQ ID NO: 7, SEQ ID NO: 8, and an adjuvant.

In certain embodiments, the composition comprises a plurality of leptospira peptides, wherein the plurality of leptospira peptides comprises a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6. In some embodiments, the composition consists essentially of SEQ ID NO: 3, SEQ ID NO: 4, SEQ ID NO: 5, and SEQ ID NO: 6. In some embodiments, the composition consists essentially of SEQ ID NO: 3, SEQ ID NO: 4, SEQ ID NO: 5, SEQ ID NO: 6, and an adjuvant.

### Protein production

The proteins of the invention, or immunogenic fragments thereof, can be produced by any known method of producing a linear amino acid sequence, such as recombinant DNA techniques. A nucleic acid sequence which encodes a protein or peptide of the invention, or a multimer of the said peptides, is introduced into an expression vector. Suitable expression vectors are for instance plasmids, cosmids, viruses and YAC (yeast artificial chromosome) which comprise necessary control regions for replication and expression. The expression vector may be stimulated to expression in a host cell. Suitable host cells are for example bacteria, yeast cells and mammalian cells. Such techniques are well known in the art and described for instance by Sambrook et al., Molecular Cloning: A Laboratory Manual, Cold Spring Harbor Laboratory Press, Cold Spring Harbor, 1989. Other well-known techniques are degradation or synthesis by coupling of one amino acid residue to the next one in liquid phase or preferably on a solid phase (resin) for instance by the so- called Merrifield synthesis. See for instance Barany and Merrifield in the Peptides, Analysis, Synthesis, Biology, Vol.2, E. Gross and Meinhofer, Ect. (Acad. Press, N.Y., 1980), Kneib-Coronier and Mullen Int. J. Peptide Protein Res., 30, p.705-739 (1987) and Fields and Noble Int.J. Peptide Protein Res., 35, p. 161-214 (1990).

### Protein purification

In some embodiments, the proteins of SEQ ID NO:1 to SEQ ID NO: 8, or immunogenic fragments thereof, are purified from contaminants. The contaminants can be cellular proteins remaining after cloning and expression of the proteins of interest in cell systems, or chemicals remaining after chemical synthesis of proteins or fragments thereof. Suitable methods to purify proteins or peptides from a mixture of contaminants comprise, but are not limited to, dialysis, gel filtration chromatography, anion exchange chromatography, cation exchange chromatography, affinity chromatography, or hydrophobic interaction chromatography such as C4 or C18 chromatography in conjunction with high performance liquid chromatography (HPLC). The purity of the proteins can be assessed by methods including, but not limited to, SDS polyacrylamide gel electrophoresis (PAGE), native PAGE, mass spectrometry, HPLC, FPLC and immunoblotting. In certain embodiments, the purity of the peptide(s) of the invention is at least 80%, at least 85%, at least 90%, at least 95%, at least 96%, at least 97%, at least 98%, at least 99%, or 100%.

In some embodiments, the invention provides mRNA molecules encoding one or more of the proteins of SEQ ID NO:1 to SEQ ID NO: 8, or mRNA encoding for immunogenic fragments thereof. In some embodiments, the mRNA is chemically modified to increase stability.

### Pharmaceutical Compositions and Formulations

The vaccine of the invention may be formulated as a pharmaceutical composition. In some embodiments, the vaccine comprises one or more purified or isolated proteins from SEQ ID NO:1 to SEQ ID NO: 8.

A pharmaceutical composition may be in a form suitable for administration to a subject (i.e. mammal), and the pharmaceutical composition may further comprise one or more pharmaceutically acceptable carriers, one or more additional ingredients, or some combination of these. The various components of the pharmaceutical composition may be present in the form of a physiologically acceptable salt, such as in combination with a physiologically acceptable cation or anion, as is well known in the art.

In one embodiment, the pharmaceutical compositions useful for practicing the method of the invention may comprise an adjuvant. Non-limiting examples of suitable adjuvants are Freund's complete adjuvant, Freund's incomplete adjuvant, AddaVax^{™}, Quil A, Detox, ISCOMs, squalene, MPLA, and CpG or other activators of TLR or inflammasome. The pharmaceutical composition or vaccine composition can comprise any one or more of the adjuvants described herein.

Pharmaceutical compositions that are useful in the methods of the invention may be suitably developed for inhalation, oral, rectal, vaginal, parenteral, topical, transdermal, pulmonary, intranasal, buccal, ophthalmic, intrathecal, intravenous or another route of administration. Other contemplated formulations include projected nanoparticles, liposomal preparations, resealed erythrocytes containing the active ingredient, and immunologically-based formulations. The route(s) of administration is readily apparent to the skilled artisan and depends upon any number of factors including the type and severity of the disease being treated, the type and age of the veterinary or human patient being treated, and the like.

Although the descriptions of pharmaceutical compositions provided herein are principally directed to pharmaceutical compositions suitable for ethical administration to humans, it is understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation.

The composition of the invention may comprise a preservative from about 0.005% to 2.0% by total weight of the composition. The preservative is used to prevent spoilage in the case of exposure to contaminants in the environment.

### Kits

In some embodiments a kit is provided for treating, preventing, or ameliorating a given disease, disorder or condition, or a symptom thereof, as described herein wherein the kit comprises: a) compositions as described herein; and optionally b) an additional agent or therapy as described herein. The kit can further include instructions or a label for using the kit to treat, prevent, or ameliorate the disease, disorder or condition. In yet other embodiments, the invention extends to kits assays for a given disease, disorder or condition, or a symptom thereof, as described herein. Such kits may, for example, contain the reagents from PCR or other nucleic acid hybridization technology (microarrays) or reagents for immunologically based detection techniques (e.g., ELISpot, ELISA).

### Methods

In another aspect, the invention provides a method of conditioning an immune response against leptospirosis in a subject, the method comprising administering to the subject an effective amount of a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8. Short lived antibody responses may be maintained over time by repeat administration of *Leptospira* proteins to boost the immune response, such as through repetitive administrations of the proteins of SEQ ID NO: 1 to SEQ ID NO: 8. In one embodiment, a mixture of proteins comprising SEQ ID NO: 3, 4, 5, 6 ("OMP3") is used to immunize a subject, and to boost a subject. The antibodies generated may confer protection against infection by a homologous pathogen, i.e. the strain used for immunization (i.e. *Leptospira* serovar Copenhageni), or a heterologous pathogen, such as a different *Leptospira* bacteria, in the subject or a new subject when transferred. The antibodies generated can be of any class, such as IgG, IgM, or IgA or any subclass such as IgGl, IgG2a, and other subclasses known in the art.

In certain embodiments, the composition comprises a plurality of leptospira peptides, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4. In some embodiments, the composition consists essentially of SEQ ID NO: 1, SEQ ID NO: 2, SEQ ID NO: 3, and SEQ ID NO: 4. In some embodiments, the composition consists essentially of SEQ ID NO: 1, SEQ ID NO: 2, SEQ ID NO: 3, SEQ ID NO: 4, and an adjuvant.

In certain embodiments, the composition comprises a plurality of leptospira peptides, wherein the plurality of leptospira peptides comprises a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8. In some embodiments, the composition consists essentially of SEQ ID NO: 5, SEQ ID NO: 6, SEQ ID NO: 7, and SEQ ID NO: 8. In some embodiments, the composition consists essentially of SEQ ID NO: 5, SEQ ID NO: 6, SEQ ID NO: 7, SEQ ID NO: 8, and an adjuvant.

In certain embodiments, the composition comprises a plurality of leptospira peptides, wherein the plurality of leptospira peptides comprises a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6. In some embodiments, the composition consists essentially of SEQ ID NO: 3, SEQ ID NO: 4, SEQ ID NO: 5, and SEQ ID NO: 6. In some embodiments, the composition consists essentially of SEQ ID NO: 3, SEQ ID NO: 4, SEQ ID NO: 5, SEQ ID NO: 6, and an adjuvant.

Antigens that stimulate an immune response, yet do not produce pathogenic disease in a subject, are exemplary vaccine candidates. Included in the methods of the invention are immunogenic *Leptospira* proteins, or immunogenic fragments thereof that can stimulate an immune response.

The methods also include administering an adjuvant, separately or in tandem with the compositions, such as an oil-in-water emulsion, a saponin, a cholesterol, a phospholipid, a CpG, a polysaccharide, variants thereof, and a combination thereof, with the composition of the invention.

In various embodiments, the composition is administered to the subject in three doses. In various embodiments, the subject is a mammal. In various embodiments, the subject is a human.

Pharmaceutical formulations that are useful in the methods of the invention may be suitably developed for inhalational, oral, parenteral, pulmonary, intranasal, intravenous or another route of administration. Other contemplated formulations include projected nanoparticles, liposomal preparations, and immunologically-based formulations. The route(s) of administration will be readily apparent to the skilled artisan and will depend upon any number of factors including the type and severity of the disease being treated, the type and age of the veterinary or human patient being treated, and the like.

The pharmaceutical formulations described herein may be prepared by any method known or hereafter developed in the art of pharmacology. In general, such preparatory methods include the step of bringing the cells into association with a carrier or one or more other accessory ingredients, and then, if necessary or desirable, shaping or packaging the product into a desired single- or multi-dose unit.

In one embodiment, the cells of the invention are formulated using one or more pharmaceutically acceptable excipients or carriers. In one embodiment, the pharmaceutical formulations of the cells of the invention include a therapeutically effective amount of the cells of the invention and a pharmaceutically acceptable carrier. Pharmaceutically acceptable carriers, which are useful, include, but are not limited to, glycerol, water, saline, ethanol and other pharmaceutically acceptable salt solutions such as phosphates and salts of organic acids. Examples of these and other pharmaceutically acceptable carriers are described in Remington's Pharmaceutical Sciences (1991, Mack Publication Co., New Jersey).

### Administration/Dosing

In the clinical settings, delivery systems for the compositions described herein can be introduced into a subject by any of a number of methods, each of which is familiar in the art. For instance, a pharmaceutical formulation of the composition can be administered by inhalation or systemically, e.g. by intravenous injection.

The regimen of administration may affect what constitutes an effective amount. The therapeutic formulations may be administered to the subject either prior to or after the manifestation of symptoms associated with the disease or condition. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the composition of the present invention to a subject, preferably a mammal, more preferably a human, may be carried out using known procedures, at dosages and for periods of time effective to treat a disease or condition in the subject. An effective amount of the composition necessary to achieve a therapeutic effect may vary according to factors such as the extent of implantation; the time of administration; the duration of administration; other drugs, compounds or materials used in combination with the composition; the state of the disease or disorder; age, sex, weight, condition, general health and prior medical history of the subject being treated; and like factors well-known in the medical arts. Dosage regimens may be adjusted to provide the optimum therapeutic response. For example, several divided doses may be administered daily or the dose may be proportionally reduced as indicated by the exigencies of the therapeutic situation. One of ordinary skill in the art would be able to study the relevant factors and make the determination regarding the effective amount of the composition without undue experimentation.

Actual dosage levels in the pharmaceutical formulations of this invention may be varied to achieve the desired therapeutic response for a particular subject, composition, and mode of administration, without being toxic to the subject.

### Routes of Administration

Routes of administration of the compositions of the invention include inhalational, oral, nasal, rectal, parenteral, sublingual, transdermal, transmucosal (e.g., sublingual, lingual, (trans)buccal, (trans)urethral, vaginal (e.g., trans- and perivaginally), (intra)nasal, and (trans)rectal), intravesical, intrapulmonary, intraduodenal, intragastrical, intrathecal, subcutaneous, intramuscular, intradermal, intra-arterial, intravenous, intrabronchial, inhalation, and topical administration.

Suitable formulation of the composition sand dosages include, for example, dispersions, suspensions, solutions, beads, pellets, magmas, creams, pastes, plasters, lotions, discs, suppositories, liquid sprays for nasal or oral administration, aerosolized formulations for inhalation, compositions and formulations for intravesical administration and the like.

It should be understood that the formulations and compositions that would be useful in the present invention are not limited to the particular formulations set forth in the examples. The following examples are put forth so as to provide those of ordinary skill in the art with a complete disclosure and description of how to make and use the cells, differentiation methods, engineered tissues, and therapeutic methods of the invention, and are not intended to limit the scope of what the inventors regard as their invention.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples therefore, specifically point out embodiments of the present invention, and are not to be construed as limiting in any way.

### Example 1: Multi-recombinant construct as a widely-applicable vaccine against leptosprirosis

A multi-recombinant protein construct was developed herein based on biologically relevant targets identified from an attenuated-vaccine model that, when combined and used as a vaccine, elicited cross-immunity protection against death and renal colonization in the hamster model of infection.

Previous results from single recombinant vaccines using selected outer membrane/extracellular (OMP) proteins identified in the attenuated vaccine model didn't elicit significant protection against death or colonization in the hamster model, which confirms that a single protein approach for leptospirosis might not be effective. For that reason, a multi-recombinant protein approach as a vaccine against leptospirosis was demonstrated herein. Focusing on the list of 154 proteins obtained from the attenuated-vaccine model as described herein, eight OMP proteins were selected as potential candidates for a multi-recombinant protein vaccine against leptospirosis (FIG. 9 and SEQ ID NOs: 1-8). Other than the fact that those candidates are surface exposed/extracellular proteins, important characteristics for a vaccine candidate, the selection process included results regarding attenuation on the hamster model after disruption of the protein (5/8), up-regulation of transcript levels under host conditions based on growth on a dialysis membrane chamber (DMC) method in rats (3/8), and being present and conserved in all pathogenic species of *Leptospira* spp. (6/8). Four (4) non-outer membrane proteins (non-OMP) were selected to use as a control group. All selected proteins were expressed based on the genome sequence of *Leptospira* interrogans serovar Copenhageni strain Fiocruz L1-130.

The Materials and Methods used in the performance of the experiments disclosed herein are now described.

### Vaccine and challenge strains

Leptospires were cultivated in liquid EMJH medium supplemented with 1% rabbit serum. *Leptospira* interrogans serovar Copenhageni strain Fiocruz L1-130 *fcpA⁻*mutant and all the seven different strains used for the challenge experiments (Table 1) were incubated up to 7 days at 29°C, till they reached log phase (between 4 and 5 days of culture). For all immunization or infection experiments, the correct number of leptospires was determined by a Petroff-Hausser counting chamber (Fisher Scientific). The heat-killed vaccine was prepared by heat-inactivating preparations of L. interrogans strain Fiocruz L1-130 at 56°C for 20 min.

**Table 1. Leptospira strains used in this study for challenge after vaccination with L1-130 fcpA⁻ mutant**

| **Species** | **Serovar** | **Strain** | **LD₅₀^{£}** | |
|---|---|---|---|---|
| | | | **Intraperitoneal** | **Conjunctival** |
| *L. interrogans* | Copenhageni | Fiocruz | <10 | 2.15 x 10⁶ |
| | | L1-130 | | |
| | Canicola | Kito | <10 | 1.78 x 10⁷ |
| | Pomona | PO-06-047 | <10 | <10⁸ |
| | Manilae | L495 | <10 | 2.15 x 10⁷ |
| *L. kirscheri* | Grippotyphosa | RM-52 | <10³ | 5 x 10⁷ |
| *L. borgpetersenii* | Hardjo-bovis | JB197 | 10⁴ | <10⁸ |
| | Hardjo-bovis | 203* | ND | >10⁸ |

| | | | | |
|---|---|---|---|---|
| ^{£} Results are the average of two independent experiments * This strain was described to cause only kidney colonization in hamsters following intraperitoneal (IP) infection, for that reason there was no LD₅₀ for IP route. However, in two experiments the fatality rate was 25% after infection with 10⁸ leptospires by conjunctival (CJ) infection | | | | |

### Animal experimentation

### Dissemination studies

For the dissemination experiments with the Fiocruz L1-130 *fcpA⁻* mutant and L1-130 heat-killed vaccine in hamsters, a group of fifteen 3-week-old male Golden Syrian hamsters (Envigo) was inoculated subcutaneously with a dose of 10⁷ leptospires in 0.5 mL of EMJH medium. A group of three animals was euthanized at 1, 4, 7, 14, and 21 days after infection. As a control, a group of nine animals was infected with Fiocruz L1-130 WT using the same route and dose, and animals were euthanized at days 1 and 4 after infection. The final group was euthanized at onset of disease. After euthanizing the animals, blood, kidney, liver, and brain were carefully removed, collected into cryotubes, and immediately placed into liquid nitrogen before being stored at -80°C until extraction of DNA. Kidney and blood were inoculated in EMJH for culture of leptospires when necessary.

For the experiment in mice, groups of three 4-week-old female C57BL/6 mice (Jackson laboratory) were inoculated subcutaneously with different doses of the vaccine (10⁷, 10⁵, 10³, 10², and 10¹) and a control group with three animals was inoculated with Fiocruz L1-130 WT with a dose of 10⁷ leptospires. Blood was collected by retro-orbital bleeding at 1, 4, 8, 13, 15, 18, and 21 days after infection.

### Immunization and challenge

Some vaccination experiments (FIG. 2A) were performed using 3-week-old male Golden Syrian hamsters (Envigo) or 4-week-old female C57BL/6 mice, divided into groups of six to nine or four to eight animals, respectively. Animals were vaccinated with Fiocruz L1-130 *fcpA⁻* mutant using the subcutaneous route. Hamsters were vaccinated with a single dose of 10⁷ leptospires and mice were vaccinated with a range of doses (10⁷, 10⁵, 10³, 10², and 10¹) in 500 and 200 µL of EMJH medium, respectively. The heat-killed vaccine was used in a single dose of 10⁷ leptospires by subcutaneous route as a control group in hamster. In addition, groups of animals were injected with phosphate buffered saline (PBS) and served as unvaccinated controls. Blood samples were collected the day before and 20 days post-immunization by retro-orbital bleeding.

Animals were challenged on day 21 post-immunization. Hamsters were challenged by conjunctival inoculation, which mimics the natural route of infection using a lethal dose (10⁸ leptospires) of a range of serovars whose virulence has been well characterized (Table 1). Hamsters vaccinated with the heat-killed vaccine were only challenged with Fiocruz L1-130 (homologous) or Manilae L495 (heterologous) strains. Whole-cell inactivated vaccines (bacterins) against leptospirosis are known for their lack of cross-reactive protection, and for that reason only one heterologous serovar was used for comparison. Mice were challenged intraperitoneally with L. interrogans serovar Manilae L495 (10⁸ leptospires). After euthanizing the animals, kidneys were collected and stored as described above.

### Passive transfer experiments

Immune sera against Fiocruz L1-130 *fcpA⁻* mutant was generated by immunizing a group of ten 3-week-old male Golden Syrian hamsters using the same protocol, described above. A group of 10 animals injected with PBS was used to obtain control sera. Animals were euthanized at day 21 post-immunization by inhalation of CO₂. Blood was obtained by cardiac puncture, followed by separation of sera that was subsequently pooled as immune (hamster α-*fcpA*-) and control sera.

Immune or control sera were passively transferred to groups of 5 naïve female mice and seven naive male hamsters (6-7-week-old) in a dose of 0.5 and 2.0 mL, respectively, using the intraperitoneal route. After 24 hr mice and hamsters were challenged with 10⁸ leptospires of serovar Manilae L495 (heterologous strain) by intraperitoneal and conjunctival route, respectively, as described above.

### Serology

Pre- and post-vaccination sera were obtained by centrifugation of clotted blood at 1000 g for 15 min at room temperature. Sera samples were kept frozen at -20°C until analysis for the presence of antibodies against leptospires by MAT, ELISA, immunoblotting, and proteome array.

MAT was performed using standard practices and as previously described. Serum was diluted at 1:100 and tested against all the strains used in this project (Table 1). Positives samples were subsequently titrated.

For the ELISA, whole cell lysate was prepared by centrifugation of L. interrogans serovar Manilae L495 and Fiocruz L1-130 cultures (10⁸ cells) at 12,000 rpm, 4°C for 20 min. The pellets were washed twice with PBS and resuspended in 500 µL of PBS. Resuspended cultures were sonicated in ice for 6 cycles at 30 kHz with a power output of 300 W. Lysates were quantitated by Bradford assay and employed as antigen at a concentration of 150 ng/well (in 0.05 M carbonate buffer, pH 9.6). Flat-bottomed polystyrene microtiter plates (Corning) were coated with *Leptospira* antigen and incubated overnight at 4°C. The plates were washed three times with PBS-0.05% (vol/vol) Tween 20 (PBST) and incubated with blocking solution (5% blocking milk in 2% [wt/vol] bovine serum albumin) for 2 hr at 37°C. After four washes with PBST, wells were incubated with mouse immune sera, diluted 100-fold in 2% BSA, for 1 hr at 37°C. Secondary anti-mouse HRP conjugated antibody (Jackson ImmunoResearch) was used at a dilution of 50,000 (2% BSA) and incubated for 1 hr at 37°C. TMB SureBlue Reserve (SeraCare) was used for detection and the reaction was stopped by adding 100 µL of 2 N H2SO4. Absorbance (450 nm) was recorded by microplate reader (Biotek).

To evaluate the effect of proteolytic enzyme treatment on *Leptospira* antigen a previously described protocol was used. Briefly, *Leptospira* antigen coated in assay wells was treated with 0.1 mg of Proteinase K (Invitrogen) at 37°C for 2 hr. The plates were washed three times with PBST to remove unbound proteins and followed by blocking and testing as described above.

### qPCR

DNA was extracted from blood and tissue samples using the Maxwell 16 (Promega Corporation) instrument following the manufacturer's instructions. Quantitative Real-time PCR assays were performed on hamster and mouse tissues using an ABI 7500 instrument (Applied Biosystems) and Platinum Quantitative PCR Supermix-UDG (Invitrogen Corporation) with lipL32 primers and probe as described previously.

### Western blot

Immunoblots with whole cell extract of *Leptospira* strains were performed as previously described. Western blot was performed with a pool of hamster or mice immune sera *αfcpA⁻* at dilution of 1:100. For subsequent detection, HRP goat anti-mouse or anti-hamster's serum (Jackson ImmunoResearch) was employed at dilution of 1:100,000. Blots were analyzed using ChemiDoc Imager (Bio-Rad).

### Proteome array

The full ORFeome was amplified from *Leptospira* interrogans serovar Copenhageni strain Fiocruz L1-130 as previously described. The ORFs larger than 150 bp were amplified from genomic DNA, followed by recombination cloning into a T7 expression vector. Genes larger than 3 kb were cloned as segments. A list of 660 most reactive antigens were selected from previous studies with human sera of patients with leptospirosis and used for the hamster experiments. Mouse sera were tested in an array containing 330 proteins selected based on the latter. Proteins were expressed in the in vitro transcription/translation (IVTT) RTS 100 E. coli HY system (5 PRIME) and synthesized crude proteins were printed on 3-pad nitrocellulose-coated AVID slides (Grace Bio-Labs) using a Gene Machine OmniGrid 100 microarray printer (Genomic Solutions). In addition to IVTT expressed proteins, each array contained no DNA control spots consisting of IVTT reactions without the addition of a plasmid, serial dilutions of purified IgG/spots.

The arrays were probed for IgG reactivity. For serum samples, the arrays were probed at 1/100 dilution in protein array blocking buffer (GVS) supplemented with E. coli lysate (Genscript) at a final concentration of 10 mg/mL to block anti-E. coli antibodies. The arrays were incubated overnight at 4°C with constant agitation. After the overnight incubation, the arrays were washed three times with T-TBS and then incubated for 45 min at RT with biotin-conjugated anti-human IgG secondary antibody (Jackson ImmunoResearch), diluted at 1/400 in array blocking buffer, followed by Qdot 800 streptavidin conjugate (ThermoFisher Scientific). The arrays were air dried after brief centrifugation. IgG signals were detected with ArrayCam 400 s Microarray Imaging System (Grace Bio-Labs) for Q800. The array signal intensities were quantified using QuantArray software. Mean pixel intensities are corrected for local background of all spots. Protein expression was validated by microarray using monoclonal anti-polyhistidine (clone His-1, Sigma).

In addition, 30 sera (acute and convalescent) of human patients from Salvador, Brazil with confirmed acute leptospirosis were probed in the array containing 660 proteins as described above. Patient samples were collected and selected as previously described.

### Data analysis

The log¹⁰ fold change (LFC) was analyzed between pre- and post-vaccination proteome signal intensities. The chip background was subtracted and the negative values were set to one (to avoid issues taking logarithms) before calculating the LFC. Analyses were conducted on three data sets: the hamster data, which used a single attenuated-vaccine dose of 10⁷ leptospires (Hamster 10⁷), the mouse dose-response data including all vaccine doses 10¹, 10², 10³, 10⁵, and 10⁷ (Mouse all), and the subset of mice given a dose of 10⁷ leptospires of the attenuated-vaccine (Mouse 10⁷). The decision of those analysis group was made based on the fact that the high dose of the vaccine in mouse (Mouse 10⁷) was the same as in the hamster experiments (Hamster 10⁷) and was the only dose in mouse that gave 100% protection against death and colonization. Furthermore, the other doses (Mouse all) provided different levels of protection when combining death and colonization and the comparison analysis of all those three groups would increase the likelihood of identifying potential protein targets with a role in those outcomes.

Exploratory analysis of mouse data showed a dose-response relationship, with increased vaccine dose associated with increased mean signal intensity (FIG. 6) as well as decreased death and colonization. A model was used that allowed to quantify this dose-response relationship when present and to instead measure the contrast between vaccinated and unvaccinated animals if only a single dose was used. Each antigen was modeled separately. For each antigen, a linear model was fit for the LFC in each animal A as LFCA= ExperimentA+VA+LogDoseA where experiment was a factor on four levels for the mice and two levels for the hamsters. VA is an indicator variable for whether the animal A received the attenuated-vaccine or a control injection. These terms were included in all models. The LogDose term was only included in the analysis of the dose-response relationship in mice and is the logarithm of the dose (0 for control animals, 1, 2, 3, 5, and 7). The indicator variable VA prevents LogDose = 0 for control animals from being treated as a true zero. The statistic of interest was the t-statistic. For the Hamster and Mouse 10⁷ models, the VA t-statistics were interpreted, and for the Mouse All dose-response model, LogDose t-statistics were interpreted. The Benjamini-Hochberg (BHp) correction was used to control the false discovery rate at 0.05. This analysis was conducted in RStudio software (RStudio).

Prism 8 (GraphPad Software) was employed for all the statistical analysis of *in vivo* data. Fisher's exact test and analysis of variance (ANOVA) were applied to assess statistical differences between pairs of groups and multiple groups, respectively. A p-value of <0.05 was considered significant. A binomial proportion confidence interval was calculated to determine the efficiency of the vaccine in both hamster and mouse. Protein homologies of L. interrogans serovar Copenhageni strain Fiocruz L1-130 were identified by a BLAST search (http://www.ncbi.nlm.nih.gov/BLAST/). Clusters of orthologs groups (COGs), pSortB localization, transmembrane domains (TMhmm), and signal peptide (SignalP) information were obtained from Genoscope platform (http://www.genoscope.cns.fr/agc/microscope/home/). p-Value for enrichment statistical analysis was calculated using Fisher's exact test in the R environment.

**Table 2. Strains used in vaccine experiments**

| **Species** | **Serovar** | **Strain** | **LD₅₀*** |
|---|---|---|---|
| *L. interrogans* | Copenhageni | Fiocruz L1-130 | <10 |
| *L. interrogans* | Canicola | Kito | <10 |
| *L. interrogans* | Pomona | PO-06-047 | <10 |
| *L. interrogans* | Manilae | L495 | <10 |
| *L. kirschneri* | Grippotyphosa | RM52 | <10 |

| | | | |
|---|---|---|---|
| * LD₅₀ for intraperitoneal inoculation of hamsters | | | |

**Passive transfer experiments.** Golden Syrian Hamsters were immunized with one dose of the *L. interrogans* Fioruz L 1-130 *fcp1*⁻ live-attenuated vaccine, and bled the animals after 21 days to obtain hyper-immune sera. Then 3mL of the hamster sera was transferred intraperitoneally (IP) to a group of 3 naive hamsters 16 hours prior to lethal challenge of 10⁷ leptospires by IP route inwith the heterologous strain L. *interrogans* Manilae. A control group of 3 hamsters were passive-transferred with 3mL of sera from non-vaccinated animals. Animals were monitored twice daily for clinical signs of leptospirosis and death, up to 14 days post-infection. Moribund animals were immediately sacrificed by inhalation of CO₂.

**Statistical analysis.** Data were graphed and analyzed using GraphPad Prism 5.0c (GraphPad Software, La Jolla, CA). Fisher's exact test and analysis of variance (ANOVA) were performed to assess statistical significance of differences between pairs of groups and multiple groups, respectively. P values <0.05 were considered to be significant.

The Results of the experiments disclosed herein are now described.

### A motility-deficient strain as an attenuated-vaccine candidate

A previously unidentified flagellar sheath protein (*FcpA*) that was essential for translational motility, and thus for virulence, was characterized. Despite the phenotype of complete attenuation, it was observed that the L1-130*fcpA⁻* mutant caused a transient systemic infection, which was cleared 7 days after intraperitoneal inoculation of 10⁸ leptospires in hamsters. In this study, after inoculation of 10⁷ leptospires using the subcutaneous route of infection in hamsters, the presence of DNA of the mutant was detected by qPCR in all the tissues tested, with the exception of the brain (FIG. 1A). These results were similar to those observed previously, with the wild-type reaching higher number of leptospires in all tissues analyzed, leading to the death or euthanasia of the animals due to clinical signs of disease 5-7 days after infection. In comparison, the signal for the *fcpA⁻* mutant strain was undetectable after 7 days with all inoculated animals surviving with no detectable leptospires in either kidney or blood, measured by qPCR and culture. Similarly, no detectable signal was observed for the animals immunized with the L1-130 heat-killed strain (FIG. 1A). The *fcpA⁻* mutant was also tested in the mouse model using different doses of infection (FIG. 1B). Although the dose of the wild-type strain was not enough to produce disease and lethality on infected mice, all animals were colonized and the presence of the leptospiral DNA in blood was detectable until the fifteenth day after infection (FIG. 1B). Furthermore, no dose of the *fcpA⁻* mutant caused colonization (data not shown) and there was a significant difference in the magnitude of dissemination of the mutant in the blood compared to the wild type (FIG. 1B). DNA signal of the*fcpA⁻*mutant was only observed in the blood of animals infected with doses of 10⁷ and 10⁵ until the 13th and 8th day after infection, respectively. Taken together, these results indicate that although the *fcpA⁻* mutant is attenuated in both the hamster and mouse model, there is a hematogenous dissemination of this mutant, identified by detection of its DNA. The mutant appears to be cleared by the immune system before it results in observable disease or death of the animals. Furthermore, it was observed in the mouse model that the dissemination of the mutant is dose dependent. However, it is important to note that, although no signal of the mutant was seen in doses equal or lower to 10³ leptospires, the theoretic limit of detection of the qPCR assay used here is 100 leptospires/mL of blood which can result in false negative results.

### Model for cross-protective immunity to leptospirosis

It was hypothesized that the transient infection produced by the *fcpA⁻* mutant induces cross-protective responses, given previous findings. Immunization with a single dose of the *fcpA⁻* mutant (FIG. 2A) conferred complete protection against mortality in hamsters from infection with homologous and heterologous serovars (FIG. 2B and Table 3). In contrast, immunization with heat-killed leptospires conferred partial protection to the homologous but not against the heterologous serovar (FIG. 2B and Table 3). Heat-killed bacterins can give a high protection level against an homologous challenge, but usually the protocol for vaccination includes at least a second dose of the vaccine. Poor results here with the heat-killed vaccine, especially for the homologous challenge (FIG. 2B and Table 3), might be due to the lack of a vaccine boost. For the purpose of evaluating the efficacy of the attenuated-vaccine after a single dose, it was decided to keep a standard protocol for vaccination and thus using only one dose of the heat-killed vaccine as well. It is important to mention that the strain Hardjo 203 was described to cause only colonization in the hamster model infected by intraperitoneal route. However, in the LD50 experiments using the conjunctival route, a 25% death rate was reproducibly observed (Table 2). Furthermore, in the non-vaccinated group, an overall death rate of 21.4% was observed after challenge with the strain Hardjo 203, but no deaths in the vaccinated group occurred, which explains the wide 95% CI range (FIG. 2B).

Protection against renal colonization was only observed in 80% of the animals immunized with *fcpA*⁻ mutant after homologous infection. Heterologous infection gave varying levels of protection, from 0% to 35.7% (FIG. 2D and Table 3). Hamsters are highly susceptible to leptospirosis, so the finding that the attenuated strain conferred partial protection against colonization was not unexpected. To understand the efficacy of the *fcpA⁻* mutant vaccine to protect against colonization, different doses of immunization were tested using the mouse model against heterologous infection. The results indicate that the protection conferred by the *fcpA⁻* mutant is dose dependent. Against death, the vaccine conferred 100% protection up to a dose of 10³ leptospires of the *fcpA⁻* mutant (FIG. 2C and Table 4), but a dose as high as 10⁷ leptospires was necessary to obtain 100% protection against colonization (FIG. 2E and Table 4). Furthermore, quantitative analyses of renal colonization showed that, although the *fcpA⁻* mutant cannot promote complete protection, there is a significant reduction of the burden of the disease both in hamster after heterologous infection (FIG. 2F and Table 3) and in lower doses of the vaccine in the mouse model, which also revealed a dose-dependent phenotype (FIG. 2G and Table 4).

These findings indicate that a single dose of a live attenuated-vaccine elicits cross-protective immunity against serovars belonging to L. interrogans, L. kirschneri, and L. borgpetersenii, the species which encompasses the majority of serovars of human and animal health importance.

**Table 3. Efficacy of the immunization with a dose of 10⁷ leptospires of the attenuated L1-130 fcpA⁻ mutant in hamsters followed by challenge with 10⁸ leptospires with homologous or heterologous strains by conjunctival route**

| **Vaccine^{£}** | **Challenge** | | **Expt.*** | **Median days for death (range)** | | **% Vaccine protection** | | | | **% Vaccine Efficacy overall (95% CI)^{¶}** | |
|---|---|---|---|---|---|---|---|---|---|---|---|
| | | | | | | **Death (No. survivors/total)** | | **Colonization (No. negative/total)** | | | |
| | **Species** | **Serovar/Strain** | | **Vaccine** | **PBS control** | **Vaccine** | **PBS control** | **Vaccine** | **PBS control** | **Death** | **Colonization** |
| *fcpA⁻* | *L. interrogans* | Copenhageni | 1 | - | 9 (8-11) | 100 (9:9) | - (0/9) | 77.8 (7/9) | - (0/9) | 100 (89-100) | 80.6 (63.7-90.8) |
| | | Fiocruz L1-130 | 2 | - | 8 (7-9) | 100 (9/9) | - (0/8) | 55.5 (5/9) | - (0/8) | | |
| | | | 3 | - | 10 (8-11) | 100 (7/7) | - (0/7) | 100 (7/7) | - (0/7) | | |
| | | | 4 | - | 8 (8-9) | 100 (6/6) | - (0/6) | 100 (6/6) | - (0/6) | | |
| | | Manilae L495 | 1 | - | % (7-10) | 100 (9/9) | - (0/9) | 33.3 (3/9) | - (0/9) | 100 (86.7-100) | 20 (8.9-39.1) |
| | | | 2 | - | 8 (8-9) | 100 (9/9) | - (0/8) | 11 (1/9) | - (0/8) | | |
| | | | 4 | - | 9 (9-12) | 100 (7/7) | - (0/7) | 14.3 (1/7) | - (0/7) | | |
| | | Pomona | 2 | - | 10 (9-18) | 100 (8/8) | - (0/8) | 0 (0/8) | - (0/8) | 100 (80.6- 100) | 0 (0-19.4) |
| | | PO-06-047 | 3 | - | 10.5 (9-13) | 100 (8/8) | - (0/8) | 0 (0/8) | - (0/8) | | |
| | | Canicola Kito | 3 | - | 10.5 (10-15) | 100 (8/8) | - (0/8) | 37.5 (3/8) | - (0/8) | 100 (79.6-100) | 26.7 (10.9-52) |
| | | | 4 | - | 11 (9-11) | 100 (7/7) | - (0/7) | 14.3 (1/7) | - (0/7) | | |
| | *L. kirschneri* | Grippotyphosa | 3 | - | 9 (8-11) | 100 (7/7) | - (0/7) | 28.6 (2/7) | - (0/7) | 100 (78.5-100) | 34 (16.3-61.2) |
| | | RM-52 | 4 | - | 9 (8-11) | 100 (7/7) | - (0/7) | 42.9 (3/7) | - (0/7) | | |
| | *L. borgpetersenii* | Hardjo-bovis | 3 | - | 7 (6-10) | 100 (7/7) | - (0/7) | 0 (0/7) | - (0/7) | 100 (78.5 - 100) | 0 (0-21.5) |
| | | JB197 | 4 | - | 11 (9-12) | 100 (7/7) | - (0/7) | 0 (0.7) | - (0/7) | | |
| | | Hardjo-bovis | 3 | - | 12 (12) | 100 (7/7) | - (5/7)^{§} | 71,4 (5/7) | - (0/7) | 100 (43.9-100)^{§} | 35.7 (16.3-61.2) |
| | | 203 | 4 | - | 14 (14) | 100 (7/7) | - (6/7)^{§} | 0 (0/7) | - (0/7) | | |
| Heat-killed | *L. interrogans* | Copenhageni | 1 | 8 (0.58) | 9 (8-9) | 66.7 (6-9) | - (0/9) | 55.5 (5/9) | - (0/9) | 58.9 (36-78.4) | 35.3 (17.3-58.7) |
| | | Fiocruz L1-130 | 2 | 8.5 (3.37) | 8 (8-15) | 50 (4/8) | - (0/8) | 12.5 (1/8) | (0/8) | | |
| | | Manilae L495 | 1 | 9 (0.7) | 8 (8-10) | 11 (1/9) | - (0/9) | 0 (0/9) | - (0/9) | 11.8 (3.3-34.3) | 0 (0-18.4) |
| | | | 2 | 8 (1.03) | 8 (7-9) | 12.5 (1/8) | - (0/8) | 0 (0/8) | - (0/8) | | |

| | | | | | | | | | | | |
|---|---|---|---|---|---|---|---|---|---|---|---|
| ^{£} *Leptospira interrogans* serovar Copenhageni strain Fiocruz L1-130 * The experiment identification refers to the group of strains that were tested at the same time ^{¶} Calculations based on frequency of outcomes compared to PBS-immunized animals ^{§} Only 3 14 animals were euthanized due to clinical signs within the control group | | | | | | | | | | | |

**Table 4. Efficacy of the inumunization with different doses of the attenuated L1-130 fcpA⁻ mutant in mice followed by challenge with 10³ leptospires of heterologous strain by intraperitoneal route**

| **Vaccine^{£}** | **Challenge** | | **Vaccine Dose^{£}** | **Expt.*** | **Median days for death (range)** | | **% Vaccine protection** | | | | **% Vaccine Efficacy overall (95% CI)^{¶}** | |
|---|---|---|---|---|---|---|---|---|---|---|---|---|
| | | | | | | | **Death (No. survivors/total)** | | **Colonization (No. negative/total)** | | | |
| | **Species** | **Serovar/Strain** | | | **Vaccine** | **PBS control** | **Vaccine** | **PBS control** | **Vaccine** | **PBS control** | **Death** | **Colonization** |
| *fcpA⁻* | *L. interrogans* | Manilae L495 | 10⁷ | 1 | - | 3 (3-6) | 100 (8/8) | - (0/8) | 100 (8/8) | - (0/8) | 100 (82.4-100) | 100 (82.4-100) |
| | | | | 2 | - | 3 (3) | 100 (6/6) | - (0/6) | 100 (6/6) | - (0/6) | | |
| | | | | 3 | - | 3 (3-4) | 100 (4/4) | - (0/4) | 100 (4/4) | - (0/4) | | |
| | | | 10⁵ | 2 | - | 3 (3) | 100 (4/4) | - (0/4) | 50 (2/4) | - (0/4) | 100 (67.6-100) | 50 (21.5-78.5) |
| | | | | 3 | - | 3 *(3-4)* | 100 (4/4) | - (0/4) | 50 (2/4) | - (0/4) | | |
| | | | 10³ | 2 | - | 3 (3) | 100 (4/4) | - (0/4) | 25 (1/4) | - (0/4) | 100 (67.6-100) | 25 (7.1-59.1) |
| | | | | 3 | - | 3 (3-4) | 100 (4/4) | - (0/4) | 25 (1/4) | - (0/4) | | |
| | | | 10² | 2 | 3.5 (3-4) | 3 (3) | 50 (2/4) | - (0/4) | 0 (0/4) | - (0/4) | 50 (21.5-78.5) | 0 (0-32.4) |
| | | | | 3 | 3.5 (3-4) | 3 (3-4) | 50 (2/4) | - (0/4) | 0 (0/4) | *-* (0/4) | | |
| | | | 10¹ | 2 | 3.5 (3-4) | 3 (3) | 0 (0/4) | - (0/4) | 0 (0/4) | - (0/4) | 0 (0-32.4) | 0 (0-32.4) |
| | | | | 3 | 4 (3-5) | 3 (3-4) | 0 (0/4) | - (0/4) | 0 (0/4) | - (0/4) | | |

| | | | | | | | | | | | | |
|---|---|---|---|---|---|---|---|---|---|---|---|---|
| ^{£} *Leptospira interrogans* serovar Copenhageni strain Fiocruz L 1-130 * The experiment identification refers to the group of strains that were tested at the same time ^{¶} Calculations based on frequency of outcomes compared to PBS-immunized animals | | | | | | | | | | | | |

### Antibodies against Leptospira proteins as a correlate for the cross-protective immunity

The *fcpA*⁻ attenuated-vaccine induced a weak agglutinating antibodies response to the homologous serovar, Copenhageni, and undetectable microscopic agglutination test (MAT) titers against heterologous serovars, both in hamsters (FIG. 3A) and mice (FIG. 3C). Furthermore, in the mouse model, agglutinating antibodies were only measurable with a dose of at least 10⁵ leptospires (FIG. 3C). In contrast, a single dose of the *fcpA⁻* mutant was able to induce a robust immune response against leptospiral proteins, recognizing proteins across the different species of *Leptospira* used in the hamster model (FIG. 3B) and the heterologous strain used in the mouse model with a dose of at least 10³ leptospires (FIG. 3D). In addition, the presence of detectable antibodies measured by ELISA correlates with the highest dose that induced 100% protection against death in the mouse model (10³ leptospires), and there is a decrease on the OD for all doses when the Manilae antigen was treated with proteinase K (FIG. 3E). Furthermore, passive transfer experiments using hamster-immune sera against *fcpA*⁻ attenuated-vaccine conferred 100% protection against heterologous lethal infection in hamsters (FIG. 3F) and mice (FIG. 3G). Taken together, these results indicate that anti-*Leptospira* protein antibodies, and not agglutinating antibodies, are the correlate of vaccine-mediated cross-protective immunity.

### Highly conserved seroreactive proteins as potential targets for eliciting cross-protective responses

The antibody response to the attenuated-vaccine was characterized using a downsized proteome array of 660 and 330 ORFs for hamster and mouse sera, respectively. A total of 133 (FIG. 4A) and 56 (FIG. 4B) protein targets were identified on the analysis of hamsters (Hamster 10⁷) and mice (Mouse 10⁷) respectively, immunized with a dose of 10⁷ leptospires and a total of 13 protein targets (FIG. 4C) on the analysis of mouse immunized with different doses of the attenuated-vaccine (Mouse all). The reason to analyze the mouse results separately was based on the fact that a dose of 10⁷ leptospires of the attenuated-vaccine was able to give 100% cross-protection against lethality and colonization (FIGs. 2C and 2E). When combined, these three different analyses resulted in a total of 154 unique protein targets (FIG. 4D and Table 5). Of those, 55% (85) have no prediction of localization and 23% (36), 14% (21), and 8% (12) have a prediction to be cytoplasmic membrane-associated, outer membrane proteins (OMP), and cytoplasmic, respectively (FIG. 5A). Enrichment analysis showed a 5.0-fold (p=4.51E-10) and 1.8-fold (p=2.92E-04) enrichment for OMP and cytoplasmic membrane-associated, respectively (FIG. 5B). In contrast, cytoplasmic proteins were 0.3-fold (p=2.91E-10) underrepresented in reactive antigens groups (FIG. 5B).

**Table 5. Complete list of 154 protein targets identified by the proteome array as correlates of immunity for the attenuated vaccine model.**

| **ORF ID** | **Product name** | **Gene** | **PsortB Localization** | **COG id** | **Function** | **Class ID** | **Description** | **Process** |
|---|---|---|---|---|---|---|---|---|
| LIC10215 | Hypothetical protein | - | Cytoplasmic Membrane | COG495 6 | Integral membrane protein (PIN domain superfamily) | R | General function prediction only | Poorly characterized |
| LIC104C05 | Hypothetical protein | - | Outer Membrane | COG045 7 | FOG: TPR repeat | R | General function prediction only | Poorly characterized |
| LIC10465 | Partial Ig-like repeat-containing protein | ligA 7-13 | Unknown | Unknown | Unknown | Unknown | Unknown | Unknown |
| LIC11845 | Hypothetical protein | - | Unknown | COG033 0 | Membrane protease subunits, stomatin/prohibitin homologs | O | Posttransiational modification, protein turnover, chaperones | Cellular processes and signaling |
| LIC12180 | Methyltransferase | - | Unknown | COG050 0 | SAM-dependent methyltransferases | RQ | General function prediction only | Poorly characterized |
| LIC20118 | Hypothetical protein | - | Unknown | Unknown | Unknown | Unknown | Unknown | Unknown |
| LIC20250 | Peptidoglycan-associated cytoplasmic membrane protein | - | Outer Membrane | COG136 0 | Flagellar motor protein | MN | Cell wall / membrane / envelope biogenesis | Cellular processes and signaling |
| LIC10017 | Hypothetical protein | - | Outer Membrane | Unknown | Unknown | Unknown | Unknown | Unknown |
| LIC10053 | Hypothetical protein | - | Unknown | COG045 7 | FOG: TPR repeat | R | General function prediction only | Poorly characterized |
| LIC10115 | Hypothetical protein | - | Cytoplasmic Membrane | COG165 1 | Protein-disulfide isomerase | OS | Posttranslational modification, protein turnover, chaperones | Cellular processes and signaling |
| LIC10192 | Hypothetical protein | - | Cytoplasmic | COG056 4 | Pseudouridylate synthases, 23S RNA-specific | J | Translation, ribosomal structure and biogenesis | Information storage and processing |
| LIC10484 | Partial Ig-ike repeat-containing | ligB 7-12 | Unknown | Unknown | Unknown | Unknown | Unknown | Unknown |
| | protein | | | | | | | |
| LIC10486 | Hypothetical protein | - | Unknown | Unknown | Unknown | Unknown | Unknown | Unknown |
| LIC10502 | Cytoplasmic membrane protein | - | Unknown | Unknown | Unknown | Unknown | Unknown | Unknown |
| LIC10623 | Flagellar motor protein MotB | motB | Unknown | COG136 0 | Flagellar motor protein | N | Cell motility | Cellular processes and signaling |
| LIC10624 | Flagellar protein | fliL | Unknown | COG158 0 | Flagellar basal body-associated protein | N | Cell motility | Cellular processes and signaling |
| LIC10892 | Biopolymer transport exbB-like protein | - | Cytoplasmic Membrane | COG081 1 | Biopolymer transport proteins | U | Intracellular trafficking, secretion, and vesicular transport | Cellular processes and signaling |
| LIC11352 | LipL32 | lipL32 | Unknown | Unknown | Unknown | Unknown | Unknown | Unknown |
| LIC11389 | Flagellar protein B | flbB | Unknown | COG041 9 | ATPase involved in DNA repair | L | Replication, recombination and repair | Information storage and processing |
| LIC11392 | Flagellar MS-ring protein | fliF | Unknown | COG176 6 | Flagellar biosynthesis/type III secretory pathway Lipoprotein | NU | Cell motility | Cellular processes and signaling |
| LIC11436 | Hypothetical protein | - | Outer Membrane | Unknown | Unknown | Unknown | Unknown | Unknown |
| LIC11456 | LipL31 | lipL31 | Unknown | COG119 6 | Chromosome segregation ATPases | D | Cell cyde control, cell division, chromosome partitioning | Cellular processes and signaling |
| LIC11570 | General secretory pathway protein D | gspD | Otfter Membrane | COG145 0 | Type II secretory pathway, component PulD | NU | Cell motility | Cellular processes and signaling |
| LIC 11653 | Hypothetical protein | - | Unknown | Unknown | Unknown | Unknown | Unknown | Unknown |
| LIC11955 | Hypothetical protein | - | Cytoplasmic | COG334 3 | DNA-directed RNA polymerase, delta subunit | K | Transcription | Information storage and processing |
| LIC11966 | Hypothetical protein | - | Unknown | Unknown | Unknown | Unknown | Unknown | Unknown |
| LIC11980 | See-independent protein translocase protein TatA | tatA | Unknown | COG182 6 | See-independent protein secretion pathway components | U | Intracellular trafficking, secretion, and vesicular transport | Cellular processes and signaling |
| LIC12015 | Hypothetical protein | - | Unknown | Unknown | Unknown | Unknown | Unknown | Unknown |
| LHC12277 | Hypothetical protein | - | Unknown | COG041 9 | ATPase involved in DNA repair | L | Replication, recombination and repair | Information storage and processing |
| LIC12538 | Preprotein translocase subunit SecD | secD | Cytoplasmic Membrane | COG034 2 | Preprotein translocase subunit SecD | U | Intracellular trafficking, secretion, and vesicular transport | Cellular processes and signaling |
| LIC12544 | DNA binding protein | - | Unknown | COG142 6 | Uncharacterized protein conserved in bacteria | S | Function unknown | Poorly characterized |
| LIC120866 | LipL41 | lipL41 | Unknown | COG045 7 | FOG: TPR repeat | R | General function prediction only | Poorly characterized |
| LIC13313 | Hypothetical protein | - | Unknown | Unknown | Unknown | Unknown | Unknown | Unknown |
| LHC13382 | Polysaccharide deacetylase | - | Unknown | COG424 9 | Uncharacterized protein containing caspase domain | RM | General function prediction only | Poorly characterized |
| LiC20172 | Lipoprotein | - | Outer Membrane | Unknown | Unknown | Unknown | Unknown | Unknown |
| LiC20218 | Hypothetical protein | - | Unknown | COG081 0 | Periplasmic protein TonB, links inner and outer membranes | M | Cell wall / membrane / envelope biogenesis | Cellular processes and signaling |
| LIC10464 | Partial Ig-like repeat-containing protein | ligB C-Terminal | Unknown | Unknown | Unknown | Unknown | Unknown | Unknown |
| LIC10464 | Partial Ig-like repeat-containing protein | ligB 1-6 | Unknown | Unknown | Unknown | Unknown | Unknown | Unknown |
| LIC10845 | Hypothetical protein | - | Unknown | COG485 6 | Uncharacterized protein conserved in bacteria | S | Function unknown | Poorly characterized |
| LIC10870 | Hypothetical protein | - | Unknown | Unknown | Unknown | Unknown | Unknown | Unknown |
| LIC11886 | Putative Lipoprotein | - | Unknown | COG201 0 | Cytochrome c, mono- and diheme variants | C | Energy production and conversion | Metabolism |
| LIC10171 | Hypothetical protein | - | Unknown | Unknown | Unknown | Unknown | Unknown | Unknown |
| LIC11206 | Hypothetical protein | - | Cytoplasmic Membrane | Unknown | Unknown | Unknown | Unknown | Unknown |
| LIC20109 | Hypothetical protein | - | Cytoplasmic Membrane | Unknown | Unknown | Unknown | Unknown | Unknown |
| LiC10210 | Cytochrome/quinol oxidase subunit 3 | cyoC | Cytoplasmic Membrane | COG184 5 | Heme/copper-type cytochrome/quinol oxidase, subunit 3 | C | Energy production and conversion | Metabolism |
| LIC10544 | Outer membrane protein | - | Unknown | Unknown | Unknown | Unknown | Unknown | Unknown |
| LIC10588 | Hypothetical protein | - | Cytoplasmic Membrane | COG220 8 | Serine phosphatase RsbU, regulator of sigma subunit | KTG | Transcription | Information storage and processing |
| LiC10945 | Hypothetical protein | - | Unknown | Unknown | Unknown | Unknown | Unknown | Unknown |
| LIC11271 | Hypothetical protein | - | Unknown | Unknown | Unknown | Unknown | Unknown | Unknown |
| LIC11742 | Magnesium transporter | mgtE | Cytoplasmic Membrane | COG223 9 | Mg/Co/Ni transporter MgtE (contains CBS domain) | P | Inorganic ion transport and metabolism | Metabolism |
| LIC11939 | Hypothetical protein | - | Unknown | Unknown | Unknown | Unknown | Unknown | Unknown |
| LIC12001 | Succinate dehydrogenase cytochrome subunit B | sdhC | Cytoplasmic Membrane | Unknown | Unknown | Unknown | Unknown | Unknown |
| LIC12242 | Hypothetical protein | - | Unknown | Unknown | Unknown | Unknown | Unknown | Unknown |
| LIC12493 | Putative Lipoprotein | - | Unknown | Unknown | Unknown | Unknown | Unknown | Unknown |
| LIC12537 | Preprotein | secF | Cytoplasmic | COG034 | Preprotein | U | Intracellular trafficking, | Cellular |
| | translocase subunit SecF | | Membrane | 1 | translocase subunit SecF | | secretion, and vesicular transport | processes and signaling |
| LIC12814 | Thymidylate kinase | tmk | Cytoplasmic | COG012 5 | Thymidylate kinase | F | Nucleotide transport and metabolism | Metabolism |
| LIC13012 | Hypothetical protein | - | Cytoplasmic Membrane | COG537 3 | Predicted membrane protein | SEGPR | Function unknown | **Poorly** characterized |
| LIC13479 | Peptidoglycan-associated cytoplasmic membrane protein | - | Outer Membrane | COG288 5 | Outer membrane protein and related peptidoglycan-associated (lipo)proteins | M | Cell wall / membrane / envelope biogenesis | Cellular processes and signaling |
| LIC20073 | Hypothetical protein | - | Cytoplasmic Membrane | Unknown | Unknown | Unknown | Unknown | Unknown |
| LIC20214 | Hypothetical protein | - | Outer Membrane | Unknown | Unknown | Unknown | Unknown | Unknown |
| LIC10009 | Putative Lipoprotein | - | Unknown | Unknown | Unknown | Unknown | Unknown | Unknown |
| LIC10050 | Peptidoglycan-associated cytoplasmic membrane protein | - | Outer Membrane | COG288 5 | Outer membrane protein and related peptidoglycan-associated (lipo)proteins | MNU | Cell wall / membrane / envelope biogenesis | Cellular processes and signaling |
| LIC10054 | Putative Lipoprotein | rlpA | Unknown | COG079 7 | Lipoproteins | M | Cell wall / membrane / envelope biogenesis | Cellular processes and signaling |
| LIC10125 | Hypothetical protein | - | Unknown | COG045 7 | FOG: TPR repeat | R | General function prediction only | Poorly characterized |
| LIC10191 | Peptidoglycan associated Cytoplasmic membrane protein | - | Unknown | COG288 5 | Outer membrane protein and related peptidoglycan-associated (lipo)proteins | M | Cell wall / membrane / envelope biogenesis | Cellular processes and signaling |
| LIC10231 | Hypothetical protein | - | Unknown | Unknown | Unknown | Unknown | Unknown | Unknown |
| LIC10314 | Hypothetical protein | - | Cytoplasmic Membrane | COG306 4 | Membrane protein involved in colicin uptake | MD | Cell wall / membrane / envelope biogenesis | Cellular processes and signaling |
| LIG10325 | Hemolysin | hlyX | Outer Membrane | COG045 7 | FOG: TPR repeat | R | General: function prediction only | Poorly characterized |
| LIC10402 | Hypothetical protein | - | Cytoplasmic | COG045 7 | FOG: TPR repeat | R | General: function prediction only | Poorly characterized |
| LIC10421 | Hypothetical protein | - | Unknown | Unknown | Unknown | Unknown | Unknown | Unknown |
| LIC10483 | Hypothetical protein | - | Unknown | COG426 0 | Putative virion core protein (lumpy skin disease virus) | S | Function unknown | Poorly characterized |
| LIC10562 | Putative Lipoprotein | - | Unknown | COG284 9 | Uncharacterized protein conserved in bacteria | S | Function unknown | Poorly characterized |
| LIC10589 | Ammonium transporter | amt3 | Cytoplasmic Membrane | COG000 4 | Ammonia permease | PNT | Inorganic ion transport and metabolism | Metabolism |
| LIC10628 | Putative Lipoprotein | - | Unknown | COG201 0 | Cytochrome c, mono- and diheme variants | C | Energy production and conversion | Metabolism |
| LIC10711 | Cytoplasmic membrane protein | - | **Outer** Membrane | Unknown | Unknown | Unknown | Unknown | Unknown |
| LIC10754 | DNA-directed RNA polymerase subunit beta | rpoC | Cytoplasmic | COG008 6 | DNA-directed RNA polymerase, beta' subunit/160 kD subunit | Unknown | Transcription | Information storage and processing |
| LIC10788 | Flagellar filament sheath protein | flaA-1 | Unknown | Unknown | Unknown | Unknown | Unknown | Unknown |
| LIC10812 | Cytochrome c-type biogenesis precursor | ccmH | Unknown | COG308 8 | Uncharacterized protein involved in biosynthesis of c-type cytochromes | O | Posttranslational modification, protein turnover, chaperones | Cellular processes and signaling |
| LIC10844 | Hypothetical protein | - | Cytoplasmic Membrane | COG162 4 | Uncharacterized conserved protein | S | Function unknown | Poorly characterized |
| LIC10891 | Hypothetical protein | - | Unknown | COG084 8 | Biopolymer transport protein | U | Intracellular trafficking, secretion, and vesicular transport | Cellular processes and signaling |
| LIC10002 | Hypothetical protein | - | Unknown | Unknown | Unknown | Unknown | Unknown | Unknown |
| LIC11003 | LipL71 | lipL71 | Outer Membrane | COG134 0 | Uncharacterized archaeal coiled-coil protein | SD | Function unknown | Poorly characterized |
| LIC11008 | Hypothetical | - | Unknown | Unknown | Unknown | Unknown | Unknown | Unknown |
| | protein | | | | | | | |
| LIC11060 | Hypothetical protein | - | Cytoplasmic Membrane | COG180 8 | Predicted membrane protein | S | Function unknown | Poorly characterized |
| LIC11073 | Putative Lipoprotein | - | Unknown | Unknown | Unknown | Unknown | Unknown | Unknown |
| LIC11148 | Membrane metalloendopeptid ase | - | Unknown | COG073 9 | Membrane proteins related to metallo-endopeptidases | M | Cell wall / membrane / envelope biogenesis | Cellular processes and signaling |
| LICI1186 | Putative Flagellar protein | flbC | Outer Membrane | COG041 9 | ATPase involved in DNA repair | L | Replication, recombination and repair | Information storage and processing |
| LIC11227 | Hypothetical protein | - | Unknown | Unknown | Unknown | Unknown | Unknown | Unknown |
| LIC11245 | Hypothetical protein | - | Cytoplasmic | COG155 5 | DNA uptake protein and related DNA-binding proteins | L | Replication, recombination and repair | Information storage and processing |
| LIC11296 | dolichyl-phosphate mannose synthase | dpm | Cytoplasmic Membrane | COG046 3 | Glycosyl-transferases involved in cell wall biogenesis | MR | Cell wall / membrane / envelope biogenesis | Cellular processes and signaling |
| LIC11299 | Putative Lipoprotein | - | Cytoplasmic | Unknown | Unknown | Unknown | Unknown | Unknown |
| LIC11324 | Flagellar basal body rod protein FlgG | flgG | Unknown | COG478 6 | Flagellar basal body rod protein | N | Cell motility | Cellular processes and signaling |
| LIC11370 | Flagellar motor switch protein | fliN | Unknown | COG177 6 | Chemotaxis protein CheC, inhibitor of MCP methylation | NUT | Cell motility | Cellular processes and signaling |
| LIC11396 | Cytoplasmic membrane protein | - | Cytoplasmic Membrane | Unknown | Unknown | Unknown | Unknown | Unknown |
| LIC11486 | Hypothetical protein | - | Unknown | COG045 7 | FOG: TPR repeat | RL | General function prediction only | Poorly characterized |
| LIC11498 | Hypothetical protein | - | Unknown | COG119 6 | Chromosome segregation ATPases | D | Cell cycle control, cell division, chromosome partitioning | Cellular processes and signaling |
| LIC11569 | Hypothetical protein | - | Unknown | COG303 1 | Type II secretory pathway, component PuIC | U | Intracellular trafficking, secretion, and vesicular transport | Cellular processes and signaling |
| LIC11573 | General secretory pathway protein G | gspG | Unknown | COG216 5 | Type II secretory pathway, pseudopilin PuIG | NU | Cell motility | Cellular processes and signaling |
| LIC11612 | Hypothetical protein | - | Outer Membrane | Unknown | Unknown | Unknown | Unknown | Unknown |
| LIC11623 | Outer membrane protein | - | Outer Membrane | COG477 5 | Outer membrane protein/protective antigen OMA87 | M | Cell wall / membrane / envelope biogenesis | Cellular processes and signaling |
| LIC11686 | Hypothetical protein | - | Unknown | COG061 3 | Predicted metal-dependent phosphoesterases (PHP family) | R | General function prediction only | Poorly characterized |
| LIC11694 | TonB-dependent outer membrane receptor | - | Outer Membrane | COG162 9 | Outer membrane receptor proteins, mostly Fe transport | P | Inorganic ion transport and metabolism | Metabolism |
| LIC11713 | Motility protein B | - | Unknown | COG136 0 | Flagellar motor protein | N | Cell motility | Cellular processes and signaling |
| LIC11753 | ExoPolysaccharíd e production protein | - | Cytoplasmic Membrane | COG330 7 | Lipid A core - O-antigen ligase and related enzymes | M | Cell wall / membrane / envelope biogenesis | Cellular processes and signaling |
| LIC11833 | Hypothetical protein | - | Cytoplasmic Membrane | COG409 5 | Uncharacterized conserved protein | s | Function unknown | Poorly characterized |
| LIC11846 | Flagellar motor switch protein | - | Unknown | COG188 6 | Flagellar motor switch/type III secretory pathway protein | NUQ | Cell motility | Cellular processes and signaling |
| LIC11940 | Heavy metal efflux pump | - | Unknown | COG084 5 | Membrane-fusion protein | M | Cell wall / membrane / envelope biogenesis | Cellular processes and signaling |
| LIC11941 | Heavy metal efflux pump | czcC | Outer Membrane | COG153 8 | Outer membrane protein | MU | Cell wall / membrane / envelope biogenesis | Cellular processes and signaling |
| LIC11959 | Hypothetical protein | - | Outer Membrane | Unknown | Unknown | Unknown | Unknown | Unknown |
| LIC11972 | Hypothetical protein | - | Unknown | Unknown | Unknown | Unknown | Unknown | Unknown |
| LIC11988 | Hypothetical protein | - | Unknown | Unknown | Unknown | Unknown | Unknown | Unknown |
| LIC11990 | Cytoplasmic membrane protein | - | Outer Membrane | COG045 7 | FOG: TPR repeat | Unknown | General function prediction only | Poorly characterized |
| LIC12075 | Hypothetical protein | - | Unknown | Unknown | Unknown | Unknown | Unknown | Unknown |
| LIC12084 | Hypothetical protein | - | Unknown | Unknown | Unknown | Unknown | Unknown | Unknown |
| LIC12183 | Hypothetical protein | - | Unknown | Unknown | Unknown | Unknown | Unknown | Unknown |
| LIC12219 | Hypothetical protein | - | Unknown | COG047 7 | Permeases of the major facilitator superfamily | EGPR | Amino acid transport and metabolism | Metabolism |
| LIC12263 | Hypothetical protein | - | Unknown | Unknown | Unknown | Unknown | Unknown | Unknown |
| LIC12281 | Hypothetical protein | - | Unknown | Unknown | Unknown | Unknown | Unknown | Unknown |
| LIC12307 | ToIC superfamily outer membrane protein | - | Unknown | COG153 8 | Outer membrane protein | MU | Cell wall / membrane / envelope biogenesis | Cellular processes and signaling |
| LIC12310 | Hypothetical protein | - | Cytoplasmic Membrane | COG067 7 | ABC-type antimicrobial peptide transport system, permease component | V | Defense mechanisms | Cellular processes and signaling |
| LIC12340 | Hypothetical protein | - | Unknown | Unknown | Unknown | Unknown | Unknown | Unknown |
| LIC12348 | Hypothetical protein | - | Cytoplasmic Membrane | COG081 0 | Periplasmic protein TonB, links inner and outer membranes | M | Cell wall / membrane / envelope biogenesis | Cellular processes and signaling |
| LIC12364 | GagA | cagA | Outer Membrane | COG119 6 | Chromosome segregation ATPases | DL | Cell cycle control, cell division, chromosome partitioning | Cellular processes and signaling |
| LIC12476 | Dihydrolipoamide succinyltransferas e sucB | sucB | Cytoplasmic | COG050 8 | Pyravale/2-oxoglutarate dehydr complex, dihydrofipoamide acyltransf. (E2) comp. and related enzymes | C | Energy production and conversion | Metabolism |
| LIC12478 | Cytoplasmic membrane protein | - | Cytoplasmic Membrane | COG070 5 | Uncharacterized membrane protein (homolog of Drosophila rhomboid) | R | General function prediction only | Poorly characterized |
| LIC12500 | Chemoreceptor (methyl-accepting chemotaxis) transmembrane protein | mcpB | Cytoplasmic Membrane | COG084 0 | Methyl-accepting chemotaxis protein | NT | Cell motility | Cellular processes and signaling |
| LIC12540 | Hypothetical protein | yajC | Unknown | COG186 2 | Preprotein translocase subunit YajC | U | Intracellular trafficking, secretion, and vesicular transport | Cellular processes and signaling |
| LIC12631 | Hemolysin | - | Unknown | Unknown | Unknown | Unknown | Unknown | Unknown |
| LIC12632 | Sphingomyelinase C 1 | sph1 | Outer Membrane | Unknown | Unknown | Unknown | Unknown | Unknown |
| LIC12673 | Hypothetical protein | - | Cytoplasmic Membrane | COG100 9 | NADH:ubiquinone oxidoreductase subunit 5 (chain L)/Multisubunit Na+/H+ antiporter, MnhA subunit | PC | Inorganic ion transport and metabolism | Metabolism |
| LIC12769 | Hypothetical protein | - | Unknown | Unknown | Unknown | Unknown | Unknown | Unknown |
| LIC12854 | 50S ribosomal protein L15 | rpIO | Unknown | COG020 0 | Ribosomal protein L15 | J | Translation, ribosomal structure and biogenesis | Information storage and processing |
| LIC12921 | Methyl-accepting chemotaxis protein | mcpA | Cytoplasmic Membrane | COG084 0 | Methyl-accepting chemotaxis protein | NT | Cell motility | Cellular processes and signaling |
| LIC12952 | S-layer-like protein | - | Cytoplasmic Membrane | Unknown | Unknown | Unknown | Unknown | Unknown |
| LIC12993 | Hypothetical protein | - | Unknown | Unknown | Unknown | Unknown | Unknown | Unknown |
| LIC13019 | Hypothetical protein | - | Unknown | Unknown | Unknown | Unknown | Unknown | Unknown |
| LIC13050 | Hypothetical protein | - | Unknown | COG140 9 | Predicted phosphohydrolases | R | General function prediction only | Poorly characterized |
| LIC13084 | Hypothetical protein | - | Unknown | COG053 2 | Translation initiation factor 2 (IF-2; GTPase) | J | Translation, ribosomal structure and biogenesis | Information storage and processing |
| LIC13200 | Hypothetical protein | - | Cytoplasmic Membrane | Unknown | Unknown | Unknown | Unknown | Unknown |
| LIC13203 | Hypothetical protein | - | Cytoplasmic Membrane | Unknown | Unknown | Unknown | Unknown | Unknown |
| LiC13214 | Putative cytochrome oxidase subunit | - | Cytoplasmic Membrane | COG312 5 | Heme/copper-type cytochromea/quinol oxidase. subunit 4 | C | Energy production and conversion | Metabolism |
| LIC13314 | Hypothetical protein | - | Cytoplasmic Membrane | Unknown | Unknown | Unknown | Unknown | Unknown |
| LIC13410 | Hypothetical protein | - | Unknown | Unknown | Unknown | Unknown | Unknown | Unknown |
| LIC13427 | Response regulator | - | Unknown | COG078 4 | FOG: CheY-like receiver | T | Signal transduction mechanisms | Cellular processes and signaling |
| LIC13451 | Flagellar hook-associated protein FlqK | flgk | Unknown | COG125 6 | Flagellar hook-associated protein | N | Cell motility | Cellular processes and signaling |
| LIC13469 | UDP-3-O-[3-hydroxymyristoyl] glucosamine N-acyltransferase | lpxD | Cytoplasmic | COG104 4 | UDP-3-O-[3-hydroxymyristoyl] glucosamine N-acyltransferase | M | Cell wall / membrane / envelope biogenesis | Cellular processes and signaling |
| LIC13488 | Hypothetical protein | parB | Cytoplasmic | COG147 5 | Predicted transcriptional regulators | K | Transcription | Information storage and processing |
| LIC20016 | Hypothetical protein | - | Cytoplasmic Membrane | COG053 2 | Translation initiation factor 2 (IF-2; GTPase) | J | Translation, ribosomal structure and biogenesis | Information storage and processing |
| LIC20060 | Hypothetical protein | - | Cytoplasmic | COG050 0 | SAM-dependent methyltransferases | RQ | General function prediction only | Poorly characterized |
| LIC20077 | Polysaccharide deacetylase | - | Unknown | COG072 6 | Predicted xylanase/chitin deacetylase | G | Carbohydrate transport and metabolism | Metaboilism |
| LIC20105 | DONA binding protein | - | Cytoplasmic Membrane | COG220 7 | AraC-type DNA-binding domain-containing proteins | KEGPR | Transcription | Information storage and processing |
| LIC20141 | Protease HtpX homolog | htpX | Cytoplasmic Membrane | COG050 1 | Zn-dependent protease with chaperone function | O | Posttranslational modification, protein turnover, chaperones | Cellular processes and signaling |
| LIC20203 | Bile acid Na+ symporter | yocS | Cytoplasmic Membrane | COG038 5 | Predicted Na+-dependent transporter | R | General function prediction only | Poorly characterized |
| LEPIC0277 | Hypothetical protein | - | Cytoplasmic | COG457 5 | Uncharacterized conserved protein | s | Function unknown | Poorly characterized |

Clusters of orthologous groups (COGs) of proteins were widely represented in those targets (Table 5), with at least one protein in each of the 18 functional categories. The COGs with higher representation were general function prediction only (R), cell wall/membrane/envelope biogenesis (M), intracellular trafficking, secretion, and vesicular transport (U), and cell motility (N) with 19, 17, 16, and 14 proteins, respectively. However, in addition to the 11 protein targets assigned as function unknown (S), the vast majority of the proteins had no COG assigned (59) (FIG. 5C). Enrichment analysis identified proteins with predicted COG-U, COG-N, and COG-M function as highly enriched among the reactive antigens, by 4.9-fold (p=2.27E-07), 3.1-fold (p=8.35E-05), and 1.6-fold (p<0.05), respectively (FIG. 5D). Furthermore, proteins predicted to be involved in signal transduction mechanisms (COG-T) and in amino acid transport and metabolism (COG-E) were significantly underrepresented in reactive antigens, by 0.4-fold (p=0.016) and 0.3-fold (0.02), respectively (FIG. 5D). Taken together, the enrichment analysis validates the approach disclosed herein to identify biologically relevant protein candidates for a cross-protective vaccine.

The identified 154 proteins (FIG. 7) were narrowed down to 41 protein targets (FIG. 8) based on their relationship among the three different groups of the proteome array's analysis. Seven proteins were identified in all groups (FIG. 4D, FIG. 8 and Table 5) and 31 proteins were identified in both hamster and mouse vaccinated with a dose of 10⁷ leptospires of the attenuated-vaccine (FIG. 4D, FIG. 8 and Table 5). Furthermore, three extra proteins were identified in the group of mice immunized with different doses, two between the group of mice immunized with a dose of 10⁷ leptospires (FIG. 4D, FIG. 8 and Table 5) and one extra protein between the group of hamsters immunized with a dose of 10⁷ leptospires (FIG. 4D, FIG. 8 and Table 5). Hamster and mice immune sera were highly reactive to the majority of the 41 proteins, in contrast to the low reactivity for the control sera and animals vaccinated with the heat-killed vaccine (FIG. 8), indicating the ability of the attenuated-vaccine to induce immunity against leptospiral proteins. Plausible vaccine candidates were identified among these 41 seroreactive proteins (FIG. 8), which included six OMPs and known putative virulence factors such as LipL32, LipL41, and Lig proteins, providing supportive evidence for using proteome arrays to identify such proteins. Not surprisingly, 40% of those targets are identified as hypothetical proteins with no described function. However, the majority (70%) have high amino acid sequence identity (>80%) among their respective orthologs in all the 13 pathogenic *Leptospira* species analyzed (FIG. 8), and therefore may be targeted for eliciting cross-protective responses. Moreover, sera from confirmed patients with acute leptospirosis reacted with 17 of the 41 *Leptospira* proteins recognized by sera from animals immunized with the attenuated-vaccine (FIG. 8).

### In vivo testing of the leptospirosis protein constructs

Three different combinations of 4 different recombinant proteins - "OMP1", "OMP2" and "OMP3" groups were evaluated in hamsters. Immunogenicity and protection studies were performed as proof-of-concept using groups of four 3-month-old Syrian Hamsters. Animals were vaccinated with 40µg of each protein, totalizing 160µg of protein diluted in 100µL of PBS combined with 100µL of a squalene-based adjuvant (AddaVax^{™}, Invivogen). AddaVax^{™} is an oil-in-water nano-emulsion adjuvant, based on the MF59^{®} formula, which has been licensed in Europe for use in human vaccines against influenza virus. AddaVax^{™} promotes a more balanced Th1/Th2 response than that obtained with alum, which is the most common adjuvant used in experimental vaccines against leptospirosis. More recently AddaVax^{™} has been used on vaccines experiments on leptospirosis research. Animals were given 2 boosters with intervals of 2-weeks and challenged with a dose of 10⁸ of the homologous serovar Copenhageni strain Fiocruz L1-130 or heterologous strain L. borgpetersenii serovar Hardjo-bovis by conjunctival route. Negative control groups were vaccinated with LigA, four non-OMP proteins, or adjuvant only.

As expected, all animals vaccinated with adjuvant only and non-OMP group were euthanized with symptoms between days 8 and 9 post-challenge (FIG. 10A). Animals vaccinated with "OMP1", "OMP2" and ligA survived homologous challenge with the Copenhageni serovar (FIG. 10A). However, only hamsters vaccinated with "OMP1" or "OMP2" were protected against renal colonization (FIG. 10B).

The "OMP1" multi-recombinant construct induces protection against death in hamsters challenged with serovars Copenhageni (FIG. 11A) and Hardjo (FIG. 11B). "OMP1" only protects against renal colonization in hamsters challenged with the homologous strain (Copenhageni) (FIG. 11C) but not Hardjo (FIG. 11D). However, similar kinetics of bacterial burden on blood after challenge with Copenhageni and Hardjo indicates the potential of this approach (FIG. 11E). The "OMP1" multi recombinant construct is highly immunogenic: the immune response of the "OMP1" construct after each immunization induced a significant boost effect for all proteins. There were no significant differences between animals challenged with homologous or heterologous strains. Three doses are necessary for full proteiction (FIGs. 12A, 12B).

Animals immunized with "OMP1" and challenged with L. interrogans (serovars Copenhageni, Pomona, and Canicola), L. kirschneri (serovar Grippotyphosa) and L. Borgpetersenii (serovar Hardjo) all survived infection. Further, the animals challenged with L. interrogans were also 100% protected against renal colonization (FIG. 13).

Subsequently, "OMP3" was evaluated. "OMP3" comprises two proteins from "OMP1" and two proteins from "OMP2" group. The proteins were selected based on the levels of amino acid identity among pathogenic species. Both constructs induced protection against homologous challenge (Copenhagenii) (FIG. 14A). Further, the "OMP3" construct, but not the "OMP2" construct was able to induce cross protection against death after heterologous challenge with serovar Hardjo (FIG. 14B). After challenge with Copenhageni or Hardjo, all hamsters vaccinated with the "OMP3" construct not only survived but they showed no evidence of renal colonization (FIGs. 14C, 14D). Cross protection was further evaluated for "OMP1" and "OMP3" for various species, i.e., L. interrogans (Copenhageni, Canicola, and Pomona), L. kirschneri (Grippotyphosa), and L. borgpetersenii (Hardjo) (FIG. 16). Groups of seven animals vaccinated with both "OMP1" and "OMP3" all survived the infection (FIG. 16). Vaccination with "OMP1" alone induced cross-protection only against L. interrogans, whereas vaccination with "OMP3" induced cross-protection against all tested species and the animals were 100% protected against renal colonization (FIG. 16).

The sera of animals vaccinated with "OMP3" have antibodies with high complement dependent bactericidal activity (50-60%). The killing activity is evident also with heterologous strains representing major species of *Leptospira* spp. (FIG. 15). The sera of animals vaccinated with "OMP1" and "OMP2" also have antibodies with high complement dependent bactericidal activity, albeit not as high as that for animals vaccinated with "OMP3" (FIG. 17). The anti-"OMP3" sera was further tested and showed high (40-50%) bactericidal activity against P1 and P2 of *Leptospira* spp. strains for which animal experiments cannot be conducted (FIG. 18). Additionally, each immunization with "OMP" induced a significant boost effect for all proteins and whole-cell leptospires, indicating that the antibodies induced by the recombinant proteins recognize both the recombinant and native proteins (FIG. 19). These results support the use of the compositions of the invention as a universal vaccine against leptospirosis.

Overall, the results disclosed herein are the first evidence of 100% cross-protection against death and colonization for leptospirosis using recombinant proteins, indicating that with the correct targets, as herein identified, a universal vaccine against leptospirosis using recombinant proteins is feasible.

Vaccination is a proven method for interrupting spill-over infections from livestock to humans and is the primary method to control or prevent leptospirosis in livestock animals reducing the impact of leptospirosis for improving animal production. However, there is no effective control for leptospirosis and safe and efficacious vaccines are not available for human use. Although China and Cuba produce bacterin-based vaccines for human use, there are significant concerns with their efficacy and safety, and these vaccines have not been licensed outside of their respective countries. Whole-cell vaccines are widely used for veterinary purposes but have significant limitations, since immunity is serovar-specific and of short duration. Currently available monovalent commercial vaccines are efficacious in preventing clinical disease but not all can provide protection against urinary shedding, and they can't protect across leptospires serovars. Multivalent vaccines can be efficacious in preventing clinical disease from different serovars but fail to prevent renal colonization and kidney pathology. Further, those multivalent vaccines are unable to achieve sufficient coverage against the spectrum of serovars that are important for animal and human health worldwide. Research has thus focused on characterizing promising reported candidates for a subunit vaccine, including surface-associated and host-expressed proteins. The attempts to generate an effective subunit vaccine against leptospirosis have been based on known potential virulent factors of highly expressed proteins, with no success. Different strategies have been employed aiming cross-protective immunity with renal clearance, but to date, only a few proteins have been shown to be promising protective antigens. However, protective results described are often inconsistent among different studies and difficult to reproduce depending on the strategy and adjuvants employed. Currently, no single candidate has been efficient on providing cross-protection. The attenuated-vaccine model revealed a unique list of proteins with biological relevance for cross-protective immunity against infection, and leptospirosis pathogenesis. Studies have shown that the combination of three recombinant proteins induces a more robust immune response than a vaccine based on a single component. The herein disclosed multi-recombinant protein construct, in particular "OMP3" is the first one to show evidence of 100% cross-protection against death and colonization for leptospirosis using recombinant proteins.

It is contemplated herein that the immunogenic compositions disclosed herein (e.g., comprising the multi-recombinant "OMP1", "OMP2", or "OMP3" protein constructs will be useful in a universal method of prevention against leptospirosis that will protect against all pathogenic species for *Leptospira* on different epidemiological settings around the world. The compositions of the present invention also eliminate the issues of production and safety of current bacterins available for leptospirosis, thereby being suitable for use as a human vaccine. The compositions of the present invention have the additional advantage of reduced cost for producing a leptospirosis vaccine for use in domestic and livestock animals compared to current veterinary leptospirosis vaccines.

### List of Enumerated Embodiments

Embodiment 1. A composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 2. The composition according to Embodiment 1, wherein each leptospira peptide has 90% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 3. The composition according to Embodiment 1, wherein each leptospira peptide has 95% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 4. The composition according to Embodiment 1, further comprising an adjuvant.

Embodiment 5. The composition according to Embodiment 1, further comprising a pharmaceutically acceptable carrier.

Embodiment 6. The composition according to Embodiment 1, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4.

Embodiment 7. The composition according to Embodiment 1, wherein the plurality of leptospira peptides comprises a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8.

Embodiment 8. The composition according to Embodiment 1, wherein the plurality of leptospira peptides comprises a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6.

Embodiment 9. A method of conditioning an immune response against leptospirosis in a subject, the method comprising administering to the subject an effective amount of a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 10. The method according to Embodiment 9, wherein each leptospira peptide has 90% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 11. The method according to Embodiment 9, wherein each leptospira peptide has 95% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.

Embodiment 12. The method according to Embodiment 9, wherein the composition further comprises an adjuvant.

Embodiment 13. The method according to Embodiment 9, wherein the composition further comprises a pharmaceutically acceptable carrier.

Embodiment 14. The method according to Embodiment 9, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4.

Embodiment 15. The method according to Embodiment 9, wherein the plurality of leptospira peptides comprises a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8.

Embodiment 16. The method according to Embodiment 9, wherein the plurality of leptospira peptides comprises a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6.

Embodiment 17. The method according to Embodiment 9, wherein the composition is administered to the subject in three separate doses.

Embodiment 18. The method according to Embodiment 9, wherein the subject is a mammal.

Embodiment 19. The method according to Embodiment 9, wherein the subject is a human.

### Other Embodiments

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

### CLAUSES

1. A composition comprising a plurality of leptospira peptides, each leptospira peptide having 80% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.
2. The composition according to clause 1, wherein each leptospira peptide has 90% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.
3. The composition according to clause 1, wherein each leptospira peptide has 95% or greater sequence identity to one of SEQ ID NO: 1, SEQ ID NO: 2, SEQ ID NO: 3, SEQ ID NO: 4, SEQ ID NO: 5, SEQ ID NO: 6, SEQ ID NO: 7, or SEQ ID NO: 8.
4. The composition according to clause 1, further comprising an adjuvant.
5. The composition according to clause 1, further comprising a pharmaceutically acceptable carrier.
6. The composition according to clause 1, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4.
7. The composition according to clause 1, wherein the plurality of leptospira peptides comprises a first leptospira peptide having 80% or greater sequence identity to having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8.
8. The composition according to clause 1, wherein the plurality of leptospira peptides comprises a first leptospira peptide having 80% or greater sequence identity to having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6.
9. A method of conditioning an immune response against leptospirosis in a subject, the method comprising administering to the subject an effective amount of a composition comprising a plurality of leptospira peptides, each leptospira peptide having 80% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.
10. The method according to clause 9, wherein each leptospira peptide has 90% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.
11. The method according to clause 9, wherein each leptospira peptide has 95% or greater sequence identity to one of SEQ ID NO: 1, SEQ ID NO: 2, SEQ ID NO: 3. SEQ ID NO: 4, SEQ ID NO: 5, SEQ ID NO: 6, SEQ ID NO: 7, or SEQ ID NO: 8.
12. The method according to clause 9, wherein the composition further comprises an adjuvant.
13. The method according to clause 9, wherein the composition further comprises a pharmaceutically acceptable carrier.
14. The method according to clause 9, wherein the plurality of leptospira peptides comprises a first leptospira peptide having 80% or greater sequence identity to SEQ ID NO: 1, a second leptospira peptide having 80% or greater sequence identity to SEQ ID NO: 2, a third leptospira peptide having 80% or greater sequence identity to SEQ ID NO: 3, and a fourth leptospira peptide having 80% or greater sequence identity to SEQ ID NO: 4.
15. The method according to clause 9, wherein the plurality of leptospira peptides comprises a first leptospira peptide having 80% or greater sequence identity to SEQ ID NO: 5, a second leptospira peptide having 80% or greater sequence identity to SEQ ID NO: 6, a third leptospira peptide having 80% or greater sequence identity to SEQ ID NO: 7, and a fourth leptospira peptide having 80% or greater sequence identity to SEQ ID NO: 8.
16. The method according to clause 9, wherein the plurality of leptospira peptides comprises a first leptospira peptide having 80% or greater sequence identity to SEQ ID NO: 3, a second leptospira peptide having 80% or greater sequence identity to SEQ ID NO: 4, a third leptospira peptide having 80% or greater sequence identity to SEQ ID NO: 5, and a fourth leptospira peptide having 80% or greater sequence identity to SEQ ID NO: 6.
17. The method according to clause 9, wherein the composition is administered to the subject in three separate doses.
18. The method according to clause 9, wherein the subject is a mammal.
19. The method according to clause 9, wherein the subject is a human.

## Claims

1. A composition comprising a plurality of Leptospira peptides, wherein the plurality of Leptospira peptides comprises a first Leptospira peptide having 80% or greater sequence identity to SEQ ID NO: 3, a second Leptospira peptide having 80% or greater sequence identity to SEQ ID NO: 4, a third Leptospira peptide having 80% or greater sequence identity to SEQ ID NO: 5, and a fourth Leptospira peptide having 80% or greater sequence identity to SEQ ID NO: 6.

2. The composition according to claim 1, wherein the first Leptospira peptide has 90% or greater sequence identity to SEQ ID NO: 3, the second Leptospira peptide has 90% or greater sequence identity to SEQ ID NO: 4, the third Leptospira peptide has 90% or greater sequence identity to SEQ ID NO: 5, and the fourth Leptospira peptide has 90% or greater sequence identity to SEQ ID NO: 6.

3. The composition according to claim 1, wherein the first Leptospira peptide has 95% or greater sequence identity to SEQ ID NO: 3, the second Leptospira peptide has 95% or greater sequence identity to SEQ ID NO: 4, the third Leptospira peptide has 95% or greater sequence identity to SEQ ID NO: 5, and the fourth Leptospira peptide has 95% or greater sequence identity to SEQ ID NO: 6.

4. The composition according to claim 1, further comprising an adjuvant.

5. The composition according to claim 1, further comprising a pharmaceutically acceptable carrier.

6. The composition according to claim 1, wherein the plurality of Leptospira peptides comprises a first Leptospira peptide having the amino acid sequence of SEQ ID NO: 3, a second Leptospira peptide having the amino acid sequence of SEQ ID NO: 4, a third Leptospira peptide having the amino acid sequence of SEQ ID NO: 5, and a fourth Leptospira peptide having the amino acid sequence of SEQ ID NO: 6.

7. The composition according to claim 1, wherein the plurality of Leptospira peptides consists essentially of a first Leptospira peptide having the amino acid sequence of SEQ ID NO: 3, a second Leptospira peptide having the amino acid sequence of SEQ ID NO: 4, a third Leptospira peptide having the amino acid sequence of SEQ ID NO: 5, and a fourth Leptospira peptide having the amino acid sequence of SEQ ID NO: 6.

8. The composition according to claim 1, wherein the composition consists essentially of a first Leptospira peptide having the amino acid sequence of SEQ ID NO: 3, a second Leptospira peptide having the amino acid sequence of SEQ ID NO: 4, a third Leptospira peptide having the amino acid sequence of SEQ ID NO: 5, a fourth Leptospira peptide having the amino acid sequence of SEQ ID NO: 6, and optionally an adjuvant and/or a pharmaceutically acceptable carrier.

9. A composition comprising a plurality of Leptospira peptides for use in a method of conditioning an immune response against leptospirosis in a subject, wherein the plurality of Leptospira peptides comprises a first Leptospira peptide having 80% or greater sequence identity to SEQ ID NO: 3, a second Leptospira peptide having 80% or greater sequence identity to SEQ ID NO: 4, a third Leptospira peptide having 80% or greater sequence identity to SEQ ID NO: 5, and a fourth Leptospira peptide having 80% or greater sequence identity to SEQ ID NO: 6.

10. The composition for use according to claim 9, wherein:
(a) the first Leptospira peptide has 90% or greater sequence identity to SEQ ID NO: 3, the second Leptospira peptide has 90% or greater sequence identity to SEQ ID NO: 4, the third Leptospira peptide has 90% or greater sequence identity to SEQ ID NO: 5, and the fourth Leptospira peptide has 90% or greater sequence identity to SEQ ID NO: 6; or
(b) the first Leptospira peptide has 95% or greater sequence identity to SEQ ID NO: 3, the second Leptospira peptide has 95% or greater sequence identity to SEQ ID NO: 4, the third Leptospira peptide has 95% or greater sequence identity to SEQ ID NO: 5, and the fourth Leptospira peptide has 95% or greater sequence identity to SEQ ID NO: 6.

11. The composition for use according to claim 9, wherein the composition further comprises an adjuvant, or a pharmaceutically acceptable carrier.

12. The composition for use according to claim 9, wherein the plurality of Leptospira peptides consists essentially of a first Leptospira peptide having the amino acid sequence of SEQ ID NO: 3, a second Leptospira peptide having the amino acid sequence of SEQ ID NO: 4, a third Leptospira peptide having the amino acid sequence of SEQ ID NO: 5, and a fourth Leptospira peptide having the amino acid sequence of SEQ ID NO: 6, and wherein the composition optionally further comprises an adjuvant and/or a pharmaceutically acceptable carrier.

13. The composition for use according to claim 9, wherein the composition is administered to the subject in three separate doses.

14. The composition for use according to claim 9, wherein the subject is a mammal.

15. The composition for use according to claim 9, wherein the subject is a human or wherein the subject is a canine mammal.
